# EUROPEAN PATENT SPECIFICATION

(11) **EP 4 590 221 B1**
(45) Date of publication and mention of the grant of the patent: **25.03.2026**
(21) Application number: 23768794.2
(22) Date of filing: 05.09.2023
(51) Int. Cl.: A61B 18/14, A61B 18/18, A61B 18/12, A61B 18/00

(54) **ELECTROSURGICAL INSTRUMENT AND ELECTROSURGICAL APPARATUS**
ELEKTROCHIRURGISCHES INSTRUMENT UND ELEKTROCHIRURGISCHE VORRICHTUNG
INSTRUMENT ÉLECTROCHIRURGICAL ET APPAREIL ÉLECTROCHIRURGICAL

(30) Priority: 23.09.2022 GB 202213942
(43) Date of publication of application: 30.07.2025
(73) Proprietor: Creo Medical Limited, Chepstow, Wales NP16 5UH (GB)
(72) Inventor: ULLRICH, George Christian, Chepstow, NP16 5UH (GB); JONES, Aeron, Chepstow, NP16 5UH (GB); TURNER, Louis, Chepstow, NP16 5UH (GB); HANCOCK, Christopher Paul, Chepstow, NP16 5UH (GB); JONES, Warren, Chepstow, NP16 5UH (GB); WEBB, David Edward, Chepstow, NP16 5UH (GB); THOMAS, Steven, Chepstow, NP16 5UH (GB); FITZSIMONS, Duncan James Foster, St. Albans Hertfordshire AL1 1LJ (GB)
(74) Representative: Mewburn Ellis LLP
(86) International application number: PCT/EP2023/074249
(87) International publication number: WO 2024/061613

(56) References cited:
- US-A1- 2002 107 517
- US-A1- 2008 045 947
- US-A1- 2009 234 355
- US-A1- 2021 196 357
- US-A1- 2021 196 363

## Description

### FIELD OF THE INVENTION

The invention relates to an electrosurgical instrument for sealing and/or cutting tissue. The electrosurgical instrument can be configured to grasp biological tissue and deliver microwave energy into the grasped tissue to seal the tissue by coagulation or cauterisation. The electrosurgical instrument may be used to apply pressure to close one or more blood vessels before applying electromagnetic radiation (preferably microwave energy) to seal the blood vessel(s). The electrosurgical instrument may also be arranged to cut, e.g. separate or divide, the vessel or surrounding tissue after coagulation or sealing, e.g. using radiofrequency (RF) energy and/or a mechanical cutting element, such as a blade. The invention may be applied to a vessel sealer for use in laparoscopic surgery or open surgery as well as to an endoscopic instrument.

The invention also relates to an electrosurgical apparatus for sealing and cutting tissue which comprises a generator unit for generating radiofrequency and/or microwave electromagnetic energy, and the electrosurgical instrument.

### BACKGROUND TO THE INVENTION

Electrosurgical instruments for delivering heat energy into grasped biological tissue are known. For example, it is known to deliver microwave energy from a bipolar electrode arrangement in the jaws of a forceps. The microwave energy may be used to seal a vessel by thermal denaturation of extracellular matrix proteins (e.g. collagen) within the vessel wall. The heat energy may also cauterise the grasped tissue and facilitate coagulation.

Such devices typically find application on the end of minimally invasive surgical laparoscopic tools but can equally find use in other clinical procedural areas such as gynaecology, endourology, gastrointestinal surgery, ENT procedures, or endoscopic procedures. Depending on the context of use, these devices can have differing physical construction, size, scale and complexity.

For example, a gastrointestinal instrument might be nominally of 3 mm diameter mounted on to the end of a very long flexible shaft. In contrast, a laparoscopic instrument may be used on the end of an industry standard nominal 5mm or 10mm diameter rigid or steerable steel shaft.

US 6,585,735 describes an endoscopic bipolar forceps in which the jaws of the forceps are arranged to conduct bipolar energy through the tissue held therebetween.

EP 2 233 098 describes microwave forceps for sealing tissue in which the sealing surfaces of the jaws include one or more microwave antennas for radiating microwave energy into tissue grasped between the jaws of the forceps.

WO 2015/097472 describes electrosurgical forceps in which one or more pairs of non-resonant unbalanced lossy transmission line structures are arranged on the inner surface of a pair of jaws.

US 2021/196363 A1 and US 2021/196357 A1 disclose an electrosurgical instrument comprising an end effector. The end effector comprises a first jaw and a second jaw. The first jaw comprises a first electrode. The end effector is movable from an open configuration to a closed configuration to grasp tissue.

US 2008/045947 A1 relates to an end effector assembly for use with an instrument for sealing vessels and cutting vessels. The end effector includes a pair of opposing first and second jaw members which are movable relative to one another from a first spaced apart position to a second position for grasping tissue therebetween.

US 2009/234355 A1 also relates to an end effector assembly for an electrosurgical instrument. The end effector comprises a pair of opposing first and second jaw members. At least one of the jaw members is movable relative to the other between a first open position in which the jaw members are disposed in a spaced relation relative to one another, and a second closed position in which the jaw members cooperate to grasp tissue therebetween.

US 2002/107517 A1 discloses a bipolar electrosurgical instrument having a pair of relatively moveable jaws, each of which includes a tissue contacting surface.

### SUMMARY OF THE INVENTION

The subject matter of the application is defined in the independent claims. The dependent claims describe optional embodiments of the invention.

At its most general, the present disclosure provides various types of electrosurgical instruments that can enable fine tissue cutting and dissection to be performed on tissue.

Moreover, the electrosurgical instruments may provide additional functionality, such as sealing biological tissue, such as (blood) vessels, using a confined microwave field that can yield a well-defined seal location with low thermal margin. With these additional functions, fewer device interchanges may be needed during a procedure.

The electrosurgical instruments disclosed herein may be used in any type of surgical procedure, but it is expected to find particular utility for non-invasive or minimally invasive procedures. For example, the device may be configured to be introduced to a treatment site through an instrument channel of a surgical scoping device, such as a laparoscope or an endoscope.

According to a first aspect of the present disclosure, there is provided an electrosurgical instrument for sealing and/or cutting tissue which comprises an instrument shaft, a joint, a first jaw, a second jaw, a first electrode, a second electrode, a third electrode, and a first isolating portion. The instrument shaft comprises a (coaxial) transmission line for conveying microwave electromagnetic energy and/or radiofrequency electromagnetic energy. The first jaw is attached to the instrument shaft and includes a first surface. The second jaw is attached to the instrument shaft and includes a second surface. The first, second, and/or third electrodes are configured to emit microwave and/or radiofrequency electromagnetic energy. The first isolating portion electrically isolates the first electrode from the second electrode. The first jaw and the second jaw can be moved between an open position, in which the tissue can be inserted between the first surface and the second surface, and a closed position, in which the first and second surfaces are brought together to clamp, hold, and/or grasp tissue therebetween. The first electrode and the second electrode are arranged on the first jaw. The first electrode protrudes from the first isolating portion. The third electrode is arranged on the second jaw. Optionally, the second electrode and the third electrode are arranged on opposing sides of the first electrode in the closed position and are electrically connected to each other.

The first electrode may have a dual functionality of the emitting microwave energy and radiofrequency energy. The second and/or the third electrode may act as a return electrode when the first electrode is an active electrode for radiofrequency cutting. Further, the first electrode and the second electrode can seal tissue by the emission of microwave electromagnetic energy.

In use, the electrosurgical instrument may thus perform vessel/tissue sealing and/or vessel/tissue dividing. Vessel/tissue sealing is typically the application of pressure to squash the walls of a biological vessel together, followed by the application of some form of thermal energy. In the disclosure, the thermal energy is applied by the first and second electrodes to the gripped tissue using the microwave electromagnetic energy. The pressure to the tissue can be applied by the first, second, and/or third electrodes (e.g. the first surface and second surface) and/or other parts of the first and second jaws. The applied electromagnetic energy disrupts/denatures the tissue cells and forms an amalgam of collagen predominant in vessel/tissue walls, which effectively bonds the vessel/tissue walls together. With time, post operatively, cellular recovery and regrowth occurs to reinforce the seal further. Vessel/tissue dividing is a process of cutting through a continuous biological vessel/tissue to separate it into two pieces. It is normally performed after a vessel/tissue is first sealed. Vessel/tissue dividing is performed by the first electrode. The vessel/tissue dividing occurs at the same position as vessel/tissue sealing.

The second electrode and the third electrode may each provide half-shells for containing the microwave energy emitted by the first electrode therein. The first electrode may be sandwiched between or (completely) surrounded by the second electrode and the third electrode in a closed position. The second jaw may include a second isolating portion which electrically isolates the first electrode from the third electrode in the closed position.

The first electrode may be a ridge or bar made from an electrically conductive material. The first electrode protrudes from the first isolating portion. This means that the first electrode is not flush with the first isolating portion. The second jaw may come into contact with the first electrode but not with the first isolating portion. This results in that tissue clamped between the first jaw and the second jaw is compressed more at the first electrode compared to the first isolating portion. This may be helpful for locally increasing the pressure for radiofrequency cutting at the location of the radiofrequency cutting.

Herein, the terms "proximal" and "distal" refer to the ends of the electrosurgical instrument, the shaft, and/or the coaxial transmission line further from and closer to a treatment site respectively. Thus, in use the proximal end is closer to a generator unit for providing the RF and/or microwave energy, whereas the distal end is closer to the treatment site, i.e. the patient.

The term "conductive" is used herein to mean electrically conductive, unless the context dictates otherwise.

The term "longitudinal" used below refers to the direction along the instrument channel parallel to the axis of the coaxial transmission line. The term "lateral" refers to a direction that is perpendicular to the longitudinal direction. The term "inner" means radially closer to the centre (e.g. axis) of the instrument channel. The term "outer" means radially further from the centre (axis) of the instrument channel.

The term "electrosurgical" is used in relation an instrument, apparatus or tool which is used during surgery and which utilises radiofrequency (RF) electromagnetic (EM) energy and/or microwave EM energy. Herein, RF EM energy may mean a stable fixed frequency in a range 10 kHz to 300 MHz, preferably in a range from 100 kHz to 5MHz, and more preferably in a range from 360 to 440 kHz. The microwave EM energy may mean electromagnetic energy having a stable fixed frequency in the range 300 MHz to 100 GHz. The RF EM energy should have a frequency high enough to prevent the energy from causing nerve stimulation. In use, the magnitude of the RF EM energy and the duration for which it is applied may be selected to prevent the energy from causing tissue blanching or unnecessary thermal margin or damage to the tissue structure. Preferred spot frequencies for the RF EM energy include any one or more of: 100 kHz, 250 kHz, 400 kHz, 500 kHz, 1 MHz, 5 MHz. Preferred spot frequencies for the microwave EM energy include 915 MHz, 2.45 GHz, 5.8 GHz, 14.5 GHz, 24 GHz. 2.45 GHz and/or 5.8 GHz may be preferred.

The microwave electromagnetic energy and the radiofrequency electromagnetic energy may be conveyed along a common signal pathway through the instrument shaft. For example, a coaxial cable may provide the common signal pathway for conveying both the microwave energy and the radiofrequency energy. In this arrangement, the transmission line may comprise an inductive filter for blocking the microwave energy from the cutting element, and a capacitive filter for blocking the radiofrequency energy from the first and second electrodes. In an alternative arrangement, the radiofrequency energy and microwave energy are conveyed along separate pathways within the instrument shaft (the transmission line includes separate pathways), wherein the inductive filter and capacitive filter are provided at a proximal end of the instrument shaft, e.g. in a handle. For example, a coaxial cable is provided for conveying the microwave electromagnetic energy while two or more wires are provided for conveying the radiofrequency electromagnetic energy.

The instrument shaft may be dimensioned to fit within an instrument channel of a surgical scoping device. The surgical scoping device may be a laparoscope or an endoscope. Surgical scoping devices are typically provided with an insertion tube that is a rigid or flexible (e.g. steerable) conduit that is introduced into a patient's body during an invasive procedure. The insertion tube may include the instrument channel and an optical channel (e.g. for transmitting light to illuminate and/or capture images of a treatment site at the distal end of the insertion tube). The instrument channel may have a diameter suitable for receiving invasive surgical tools. The diameter of the instrument channel may be equal to or less than 13 mm, preferably equal to or less than 10 mm, and more preferably, especially for flexible insertion tubes, equal to or less than 5 mm.

The instrument shaft and the transmission line may be flexible so that they can be inserted into the instrument channel of the scoping device. Further, the transmission line may be arranged within a lumen of the shaft. The instrument shaft may cover and/or shield the transmission line. The transmission line may extend from a distal end to a proximal end of the electrosurgical instrument. In particular, the transmission line electrically connects the first electrode and the second electrode to the generator unit.

The electrosurgical instrument discussed herein may find applicability in other tissue welding techniques. For example, the energy delivery structure may be used as an alternative to staples. In some abdominal procedures, staple guns are used to deliver 50 to 100 small staples that are fired simultaneously between jaws that can have a length of 70 mm or more, or from an annular jawed arrangements with diameters of 20 to 50 mm. In this type of application multiple antenna structures such as those discussed herein may be used to cover the required length. The antenna structures may be arranged in any number of array forms to be activated simultaneously, sequentially or progressively in a suitable manner.

The first jaw and/or the second jaw may be movable relative to their instrument shaft. The first jaw and/or the second jaw may be attached to the instrument shaft via the joint or a hinge. The joint may include a pivot axis around which the first jaw and/or the second jaw may rotate. The first jaw and/or the second jaw may be activated by one or more actuation rods or control wires respectively connected to the first jaw and/or the second jaw. The one or more actuation rods or control wires may extend within the instrument shaft to a proximal end of the electrosurgical instrument. The one or more actuation rods may be connected to a handle with which the first and/or second jaws can be actuated, e.g. opened and/or closed. The electrosurgical instrument comprises an actuation mechanism which converts a back-and-forth movement of the actuation rod(s) or control wire(s) into a rotational movement of the first jaw and/or the second jaw.

For example, both jaws can be movable, e.g. rotatable around a (common) pivot axle. In another embodiment, one of the jaws is fixed to the shaft and the other jaw is movable relative to the one jaw.

In the open position, the first jaw and the second jaw are (maximally) spaced apart so that there is a free space between the first surface of the first jaw and the second surface of the second jaw. In this way, tissue can be inserted between the first surface and the second surface in the open position. Usually, the first jaw and the second jaw are moved towards the tissue such that the tissue is pushed into the space between the first surface and the second surface in the open position of the first jaw and the second jaw.

By moving the first jaw and/or the second jaw from the open position to the closed position, the tissue between the first surface and the second surface can be grasped and/or clamped between the first surface and the second surface. In this way, the tissue can be fixed between the first surface and the second surface in the closed position. The first surface and the second surface are the faces of the first jaw and the second jaw, respectively, that face each other in the open and/or closed position.

The pair of jaws may be pivotable relative to each other about the pivot axis that lies transverse to a longitudinal axis of the coaxial transmission line. In one example, the pair of jaws comprises a static jaw that is fixed relative to the instrument shaft, and a movable jaw that is pivotably mounted relative to the static jaw to open and close the gap between the opposing inner surfaces. The energy delivery structure may be disposed on the inner surface of the static jaw. In another example, both jaws are arranged to pivot with respect to the instrument shaft, e.g. in a symmetrical forceps-type or scissors-type arrangement. Relative movement of the pair of jaws may be controlled from a handle at a proximal end of the instrument shaft. A control rod or control wires may pass through the instrument shaft to operably couple an actuation mechanism on the handle to the pair of jaws.

In another example, the pair of jaws may be arranged to move relative to one another in a manner that maintains the inner surfaces thereof in an aligned, e.g. parallel, orientation. This configuration may be desirable for maintaining a uniform pressure on grasped tissue along the length of the jaws. One example of such a closure mechanism is disclosed in WO 2015/097472.

The first jaw and/or the second jaw may have a Maryland configuration. This can include that the first jaw and the second jaw are not straight but bent/curved, e.g. forming an arc or an S-shape in a side view.

In an optional embodiment, a first face of the first isolating portion and/or the second electrode and a second face of the first isolating portion and/or the second electrode are arranged on opposing sides of the first electrode, wherein the first face and the second face define an angle of less than 180° with respect to each other.

For example, the first face and the second face define an angle in the range of 100° to 180°, e.g. 120°, 150°, or 170°. The inclined configuration of the first face and the second face increases the exposure of the first electrode so that the pressure of the first electrode in the closed position on the clamped tissue is further increased.

The first face may be (or solely include) a section of the first isolating portion that is exposed on the first surface on one side of the first electrode, and the second face may be (or solely include) a section of the first isolating portion that is exposed on the first surface on another side of the first electrode. In this embodiment, the first face and the second face do not include sections of the second electrode that are exposed on the first surface.

The exposed section of the first isolation portion (i.e. the first face and/or the second face) may be flat or planar. The first electrode may be arranged at that line where the first face and the second face would meet. Further, the first face and the second face may be symmetrical about the first electrode. Sections of the second electrode that are exposed on the first surface may be flush with the first face and/or the second face, respectively.

Alternatively, the first face and the second face may include sections of the first isolating portion and the second electrode that are exposed on the first surface. Further alternatively, the first face and the second face may only include sections of the second electrode that are exposed on the first surface. In this embodiment, the first face and the second face do not include sections of the first isolating portion that are exposed on the first surface.

In an optional embodiment, the second surface includes a third face and a fourth face. Optionally, the first face is parallel to the third face and the second face is parallel to the fourth face. Alternatively, the angle defined by the first face and the second face may be smaller than an angle defined by the third face and the fourth face. Further alternatively, the third face and the fourth face may define an angle of 180° with each other.

The third face and the fourth face may be surface areas of the second surface which may be equivalents to the first face and the second face, respectively. For example, the third face has the same surface area as the first face, and the fourth face has the same surface area as the second face. In the closed position, the first face and the second face partially or completely contact the third face and the fourth face, respectively. In the closed position, the first electrode may contact a line on the second surface which separates the third face from the fourth face.

For example, the third face and the fourth face define an angle in the range of 100° to 180°, e.g. 120°, 150°, or 170°.

The third face may include sections of the second isolating portion and/or the third electrode that are exposed on the second surface, and the fourth face may include sections of the second isolating portion and/or the third electrode that are exposed on the second surface.

The exposed section of the second isolation portion and/or the third electrode (i.e. the third face and/or the fourth face) may be flat. Further, the third face and the fourth face may be symmetrical to each other. Sections of the third electrode that are exposed on the second surface may be flush with the sections of the second isolation portion that are exposed on the second surface, respectively.

The first, second, third, and/or fourth faces may each be flat or straight surfaces.

In the embodiment of the parallel arrangement of the first face to the third face and of the second face to the fourth face, the first face may completely contact or cover the third face in a closed position and the second face may completely contact or cover the fourth face in the closed position. Thus, pressure on the tissue between the first face and the third face as well as the second face and the fourth face in the closed position may be constant along the respective surface areas. The pressure on the tissue that is applied by the first electrode is increased compared to the pressure applied by the first to fourth face due to the parallel arrangement of the respective faces.

In the embodiment in which the angle defined by the first face and the second face is smaller than the angle defined by the third face and fourth face, the pressure applied to the tissue by the respective faces decreases from the first electrode outwards (e.g. perpendicular to the longitudinal direction of the first jaw) since a gap between the first face and the third face as well as between the second face and the fourth face in the closed position increases with increased distance from the first electrode. In this configuration, pressure on the tissue can be increased or concentrated on the area of the first electrode or, in other words, in the area where radiofrequency is delivered.

In the embodiment in which the third face and the fourth face define an angle of 180°, the third face and the fourth face may define a flat or straight surface. For example, the second surface may be flat. Here again, the pressure applied on the tissue by the respective faces decreases from the first electrode outwards since a gap between the first face and the third face as well as between the second face and the fourth face in the closed position increases with increased distance from the first electrode.

In an optional embodiment, the first surface includes a (minimal or smaller) first curvature in a cross-sectional view of the first jaw and the second surface includes a second (maximal) curvature in a cross-sectional view of the first jaw, wherein the second (maximal) curvature is smaller than the first (minimal or larger) curvature.

In this embodiment, the first surface and/or the second surface are not flat or include flat surface areas (e.g. the first to fourth faces) but form curved surfaces. The curvature of the first and the second surfaces is the amount by the first surface and the second surface, respectively, deviates from being a plane in a cross-sectional view. The first and/or second curvatures may be constant (at any cross-section along the longitudinal direction of the first and/or second jaw) so that the first surface and/or the second surface may have a shape of section of a lateral surface of a cylinder. The first and/or second curvatures may vary (i.e. may not be constant) when moving away from the first electrode (at any cross-section along the longitudinal direction of the first and/or second jaw). For example, the curvature of the first surface may be smaller close to the first electrode and greater further away from the first electrode. Depending on the curvature of the second surface, the gap between the first surface and the second surface in the closed position may be constant or varies along a distance away from the first electrode (e.g. perpendicular to the longitudinal direction of the first jaw). So, the curvatures of the first surface and the second surface can be used for setting the pressure applied to tissue similar to the inclinations of the first to fourth faces.

The first curvature and the second curvature may be constant along the longitudinal direction of the first jaw and the second jaw, respectively. Alternatively, the first curvature and the second curvature vary along the longitudinal direction of the first jaw and the second jaw, respectively. For example, the curvatures of the first surface is in a range of first curvatures from a minimal curvature to a maximal curvature. The curvatures of the second surface is in a range of second curvatures from a minimal curvature to a maximal curvature. The range of first curvatures may not overlap with the range of second curvatures so that the maximal curvature of the second curvatures is smaller than the minimal curvature of the first curvatures.

According to the invention, the electrosurgical instrument further comprises a second isolating portion which is arranged on the second jaw.

A surface of the second isolating portion that is exposed on the second jaw may form or is part of the second surface. The second isolating portion arranged on the second jaw may be made from the same electrically non-conductive material as the first isolating portion.

In an optional embodiment, the third electrode forms the third face and the fourth face, wherein optionally the second isolating portion is arranged between the third face and the fourth face.

In this embodiment, the third face and the fourth face are formed or defined by the exposed sections of the third electrode. For example, two sections of the third electrode are exposed on the second surface which are separated by the second isolating portion. The second isolating portion may be arranged in line with the first electrode so that the second isolating portion contacts the first electrode in the closed position for providing an electrical isolation of the first electrode to the third electrode in the closed position.

The third face and the fourth face of the third electrode may have the above-described inclination with respect to each other or define the second curvature. If the first face and the second face include exposed sections of the first isolating portion and the first face extends parallel to the third face as well as the second face extends parallel to the fourth face in the closed position, the third face and the fourth face can contact exposed sections of the first isolating portion, i.e. the first face and the second face, respectively. This may be used for providing microwave sealing between the first face and the third face as well as between the second face and fourth face.

The second isolating portion may be made from silicon or a silicon-based material.

In an optional embodiment, the third electrode provides the second curvature, the second isolating portion being flush with or protruding from the second curvature.

The second isolating portion may protrude from the second curvature as defined by the third face and the fourth face.

Thus, in this embodiment, the second isolating portion is arranged on the section of the third electrode. So the overall shape of the second surface deviates from the second curvature in a region over which the second isolating portion extends. Alternatively, the third electrode includes a recess in which the second isolating portion is arranged. In this case, the exposed section of the second isolating portion may be flush with the exposed sections of third electrode. In this case, the complete second surface may show the second curvature.

In an optional embodiment, the second isolating portion includes a lumen and the lumen is configured to be deformed in the closed position, wherein optionally the electrosurgical instrument comprises a blocking element which is insertable into the lumen for filling the lumen.

The second isolating portion may include a flexible tube or hose. The lumen may be a channel or passage extending parallel to the first electrode in the closed position. The lumen is arranged close to the second surface such that the first electrode deforms the second isolating portion in the closed position, in particular, compresses the lumen. So in the closed position, the volume of the lumen is reduced due to the pressure applied by the first electrode compared to the open position. The lumen may be elastically deformable. The lumen may be closed or open at the distal end of the second jaw. The compression of the lumen allows to reduce the pressure that is applied on the tissue in the closed position since the deformation of the lumen results in a decrease of the pressure on the tissue.

The blocking element may include a wire, rod, and/or any other elongate body that are configured to be inserted into the lumen. The blocking element may be made from (stainless) steel or Nitinol (NiTi). The blocking element may be inserted by distally advancing the blocking element from a proximal end of the lumen to a distal end of the lumen. The blocking element may be activated by a control wire or actuation rod or forms a unitary component with the control wire or actuation rod.

If the blocking element is inserted into the lumen, the blocking element substantially fills the lumen so that the lumen can no longer be deformed upon application of pressure. The blocking element may be used for increasing the pressure on the tissue in the closed position. For example, for microwave sealing, the blocking element is not inserted into the lumen and the electrosurgical instrument is brought into the closed position. As such, the first electrode presses against the second isolating portion and reduces the volume of the lumen. This is helpful for releasing the pressure on the tissue between the second isolating portion and the first electrode. Prior to radio frequency cutting, the blocking element is inserted into the lumen so that the lumen is expanded from the compressed configuration so that additional pressure is applied to the tissue grasped between the first electrode and the second isolating portion.

In an optional embodiment, the second isolating portion and the third electrode are arranged to define a channel therebetween. Optionally, the second isolating portion is elastic and/or is made from an elastic material, such as silicon.

The channel may have the same configuration and purpose as the lumen discussed above. However, the channel is arranged between the second isolating portion and the third electrode, and not inside the second isolating portion as the lumen. As such, the channel is defined by the third electrode and the second isolating portion. The lumen may be defined only by the second isolating portion.

For example, the third electrode may include a recess extending along the longitudinal direction of the second jaw. The second isolating portion may cover this recess for forming the channel. The second isolating portion may have the configuration of a membrane so that it is deformable/elastic.

The channel may extend parallel to the first electrode in the closed position. The channel may be deformed by the first electrode in the closed position, in particular, compressed by the channel. So in the closed position, the volume of the channel is reduced due to the pressure applied by the first electrode compared to the open position. The channel may be closed or open at the distal end of the second jaw. The compression of the channel allows to reduce the pressure that is applied on the tissue in the closed position since the deformation of the lumen results in a decrease of the pressure on the tissue. Alternatively, the second isolating portion does not contact the first electrode in the closed position. For example, the second isolating portion may be positioned close to the first electrode so that the second isolating portion is not deformed in the closed position in the absence of tissue and is potentially deformed by tissue arranged between the first electrode and the second isolating portion.

In an optional embodiment, an area of the channel in a cross-sectional view of the second jaw is smaller than an area of a blocking element in a cross-sectional view of the blocking element so that the second isolating portion is deformed towards the first electrode in the closed position. The blocking element is configured to be inserted into the channel.

The blocking element may have the same features and/or optional embodiments as described above. In this embodiment, the insertion of the blocking element into the channel deforms, bends, or bulges the second isolating portion towards the first electrode in the closed position. Thus, the insertion of the blocking element can increase the pressure on the tissue between the second isolating portion and the first electrode while maintaining the first and second jaws in the closed position. This is possible as one section of the channel is formed by the rigid or non-elastic (metallic) third electrode. For example, the blocking element is pressed between the second isolating portion and the third electrode but only the second isolating portion is elastic.

The deformation or bending of the second isolating portion occurs because the blocking element would not fit into the channel when the second isolating portion is not deformed. So, the cross-sectional area of the blocking element relates to the cross-sectional area or volume that is effectively occupied by the blocking element. For example, the channel is circular having a first radius in a cross-sectional view (when no blocking element is inserted in the channel) and the blocking element is circular having a second radius in a cross-sectional view. The second radius is greater than the first radius, so the deformation of the second isolating portion results in an increase of the first radius of the channel to the second radius.

In an optional embodiment, the blocking element, which is insertable into the channel, includes a minimal diameter and a maximal diameter in a cross-sectional view of the blocking element, the channel including a first diameter, which is larger the maximal diameter of the blocking element, and a second diameter, which is greater than the minimal diameter and smaller than the maximal diameter, so that a rotation of the blocking element is configured to deform the second isolating portion towards the first electrode in the closed position.

The blocking element may have a shape in a cross-sectional view that deviates from a circle, e.g. is oval or elliptic. In a first orientation of the blocking element, the maximal diameter of the blocking element is aligned or parallel to the first diameter of the channel (the first diameter may be a maximal diameter of the channel) and the minimal diameter of the blocking element is aligned or parallel to the second diameter of the channel (the second diameter may be a minimal diameter of the channel). As the maximal diameter is smaller than the first diameter of the channel and the minimal diameter of the blocking element is smaller than the second diameter of the channel, the second isolating portion is not deformed, bent, or bulged because the blocking element fits into the channel. For deforming, bending, or bulging the second isolating portion, the blocking element is rotated so that the orientations of the first diameter and the second diameter are swapped, e.g. a rotation by 90°. In this orientation, the minimal diameter of the blocking element is aligned or parallel to the first diameter of the channel and the maximal diameter of the blocking element is aligned or parallel to the second diameter of the channel. As the maximal diameter is greater than the second diameter of the channel, the second isolating portion is deformed, bent, or bulged.

The blocking element of this configuration is rotatable and may not be movable in and out of the channel, but may remain permanently within the channel.

In an optional embodiment, the second isolating portion forms the third face and the fourth face. Optionally, the second isolating portion protrudes from the third electrode. Further, optionally only the second isolating portion is in contact with the first surface in the closed position.

Only the second isolating portion may form the third face and the fourth face. In this case, the third electrode may also be exposed at the second surface. However, the exposed sections of the third electrode are offset away from the first surface in the closed position so that the second isolating portion first comes into contact with the tissue or the first surface when moving the first jaw and the second jaw towards to the closed position. It is possible that only the second isolating portion is in contact with tissue or the first surface in the closed position, for example if the offset between the first and second faces and the exposed sections of the third electrode is large.

In this embodiment, the second isolating portion can be made from a soft flexible material such as silicon or silicon-based material. Due to the protrusion of the second isolating portion from the third electrode, pressure that is applied to the tissue in the closed position can be balanced and/or reduced.

In an optional embodiment, the electrosurgical instrument further comprises a fourth electrode. Optionally, the fourth electrode is embedded in the second isolating portion.

The fourth electrode may be configured to emit microwave energy and can be electrically connected to the first electrode either directly or via an electrical connection to the same conductor of the transmission line. In this embodiment, the second electrode may be electrically connected to third electrode. The fourth electrode may not be configured for radiofrequency cutting since it is embedded within the second isolating portion. In other words, the fourth electrode is not exposed on this second surface. Stated differently, the fourth electrode is not configured to contact tissue. Due to the provision of the third electrode, microwave energy can be emitted into the tissue from both the first jaw and the second jaw, i.e. coming from the first surface and coming from the second surface.

Any combination of silicon and other dielectric materials such as ceramics could be used as material for the second isolating portion. For example, half of a thickness of the second isolating portion could be made up of a ceramic material and the other half could be made up of a silicon material.

In this embodiment, the first face may be parallel to the third face and the second face may be parallel to the fourth face in the closed position. As the fourth electrode is embedded in the second isolating portion, the fourth electrode is under the surface of the second surface so that the fourth electrode cannot stick to tissue.

According to the invention, the second isolating portion protrudes more from the third electrode at a distal end of the second surface compared to a proximal end of the second surface.

For example, the distance between a protruding edge of the second isolating portion and an exposed section of the third electrode increases when moving from a proximal end of the second jaw towards a distal end of the second jaw. This is helpful in case the second jaw is rotatable around the same pivot axis as the first jaw. In this embodiment, proximal regions of the first surface and the second surface first contact each other compared to distal regions of the first surface and the second surface when moving from the open position to the closed position. The provision of a linear or continuous increase of the protrusion of the second isolating portion balances this undesired effect caused by the rotation of the first jaw and the second jaw around the same pivot axis.

In an optional embodiment, the second jaw includes a protruding portion and a recessed portion, wherein optionally, in the closed position, the protruding portion is in contact with the first surface and the recessed portion is spaced from the first electrode. Optionally, the protruding portion is flexible so that the second jaw can be further moved towards the first jaw relative to the closed position so that the recessed portion can be moved closer towards the first electrode.

The protruding portion and the recessed portion may have U-shape or V-shape in a cross-sectional view of the second jaw. Exposed sections of the protruding portion may define first face and the second face. The protruding portion may be provided by the third electrode and/or the recessed portion may be provided by the second isolating portion having the characteristics and/or features as described above. The protruding portion may include a silicon pad.

The protruding portion and the recessed portion may form a half-shell. The protruding portion may be configured to contact the first surface and/or the first face and the second face. The second surface may be defined by protruding portion. The recessed portion may be surrounded by the protruding portion when viewing onto the second surface. The recessed portion may be offset from the second surface for example away from the first surface in the closed position. Thus, there is a gap between the first surface/electrode and the recessed portion in the closed position. The gap can be reduced by further pressing the second jaw against the first jaw (or vice versa) so that the protruding portion is bent or compressed. In this way, the pressure on the tissue for radiofrequency cutting can be increased compared to the closed position since the recessed portion presses more against the radiofrequency electrode arranged on the first jaw compared to the closed position.

The protruding portion is sufficiently flexible so that the recessed portion can be moved further towards the first surface. The protruding portion may be bent or flexes outwards.

The protruding portion may be made from a flexible plastic material which is provided with a conductive layer for providing the shielding capabilities of the second jaw. The recessed portion may be made from a rigid material, e.g. an electrically isolating material.

In an optional embodiment, the second electrode forms a first face and a second face. Optionally, the first isolating portion is arranged between the first face and the second face.

In this embodiment, the second electrode and the first isolating portion may have to same characteristics as described above in connection with the third electrode and the second isolating portion, respectively, in which the said third electrodes provides the third face and the fourth face. In this embodiment, the fourth electrode embedded in the second isolating portion may be provided. In the closed position, microwave seals may be provided between the first and second faces of the second electrode and the third face and the fourth face of the second isolating portion. The first face may extend parallel to the third face and/or the second face may extend parallel to the fourth face.

In an optional embodiment, portions of the second electrode are plate-shaped and respectively include end faces, wherein optionally the end faces form the sections of the second electrode that are exposed on the first surface. Optionally, in a cross-sectional view of the first jaw, the portions of the second electrode are U-shaped or V-shaped.

For example, the portions of the second electrode that extend along sections of the first jaw where the first is provided may be plate-shaped. Other portions of the second electrode may have different configurations. For example, proximal and/or distal end portions of the second electrode may have shapes that deviate from a plate shape. This may be provided for forming distal and/or proximal end portions of the first jaw. End faces of the plate-shaped portions of the first electrode and/or the second electrode can be in the exposed sections on the first surface.

The plate-shaped portions of the second electrode may have a shape of the letter U, V, or variations thereof in a cross-sectional view of the first jaw. For example, the first electrode and/or the first isolating portion may be arranged within the shape defined by the U-shape or V-shape in a cross-sectional view.

The first surface may include the exposed sections of the second electrode, the exposed sections of the first isolating portion, and/or the first electrode. The first electrode and/or the second electrode are arranged within and/or on the first jaw. The first electrode and/or the second electrode are made from an electrically conductive material, such as metal, and may be connected to an inner conductor and an outer conductor of the coaxial cable, respectively.

The first electrode and the second electrode are electrically isolated from each other by the first isolating portion. Further, the first electrode and the sections of the second electrode that are exposed on the first surface are spaced apart from each other, for example by an air gap or the exposed section of the first isolating portion.

The first electrode and/or exposed sections of the second electrode may extend as lines on the first surface. In particular, the first electrode and/or the exposed sections of the second electrode define a sealing area.

The exposed sections of the second electrode and the first electrode may form (straight) lines which are separated by the gap. The exposed sections of the second electrode may form (straight) lines between which the first electrode is positioned. Exposed sections of the first isolating portion may be arranged between the (straight) lines of the first electrode and the exposed sections of the second electrode.

The sealing area is configured to emit microwave energy at the first electrode and the exposed sections of the second electrode. For example, the first electrode may be considered an active electrode and the exposed section may be considered a return electrode for radiofrequency cutting. Further, the first electrode and the exposed sections of the second electrode may be a dipole antenna for radiating microwave electromagnetic energy.

The sealing area is configured to emit microwave energy to tissue that is close to or in contact with the first electrode and the exposed sections of the second electrode. The first electrode and the exposed sections of the second electrode are arranged such that tissue that is clamped and/or grasped between first surface and the second surface in the closed position is in contact with the first electrode and the exposed sections of the second electrode or in contact with the sealing area. This means that the sealing area may have a several functionalities. They can clamp or grasp tissue (with the second surface being the counterpart), emit microwave energy, and emit radiofrequency energy.

The greatest intensity of the emitted microwave energy is achieved in a portion of the tissue that is in contact with or directly above the first electrode and the exposed sections of the second electrode. In particular, the intensity of the emitted microwave energy is highest at respective edges or corners of the first electrode and the exposed sections of the second electrode that face each other. In other words, the intensity of the emitted microwave energy is highest above the interface of the first isolating portion and the first electrode (at the first surface) and at the interface of the first isolating portion and the second electrode (at the first surface).

The first electrode is configured to cut or divide tissue that is arranged between the first electrode and the second surface. The line of the cut or divide of the tissue may be considered the cutting line. The first electrode may be arranged along the cutting line. The cutting line is arranged between the sections of the second electrode that are exposed on the first surface. Optionally, the cutting line is arranged in the middle between the sections of the second electrode that are exposed on the first surface.

The first electrode may therefore be configured to cut tissue. For example, the tissue is firstly sealed and then cut. Due to the protrusion of the first electrode from the first isolating portion, the first electrode applies more pressure on the tissue during cutting compared to the pressure applied by the first to fourth faces.

The first isolating portion may be made from an electrically non-conductive material such as a ceramic (e.g. Zirconia) or plastic material (e.g. Polyetheretherketon (PEEK)). The first isolating portion may be fixed to the first and/or the second electrode and/or vice versa. The first isolating portion may be arranged in the space formed by the U-shaped or V-shaped portions of the first electrode.

In an optional embodiment, the exposed sections of the second electrode and/or the first electrode at least partially extend parallel to each other. Optionally, the first electrode is arranged between the exposed sections of the second electrode.

The exposed sections of the second electrode completely or partially extend parallel to each other on the first surface. For example, the exposed sections are straight which are connected by a connecting section - thus forming a loop. Other shapes of the loops are possible. The first electrode may completely or partially extend parallel to the exposed section of the second electrode. In case of a loop, the second electrode forms a single section that is exposed on the first surface.

In an optional embodiment, a part of the second electrode is embedded in the first isolating portion. Optionally, a distal end portion of the second electrode is exposed on the first isolating portion.

In this embodiment, a portion of the first isolating portion completely surrounds a part of the second electrode. This part may be arranged between the proximal end of the first jaw and a distal end portion of the first jaw at which the second electrode is exposed. For example, the second electrode is exposed at the distal end face.

Further, in this embodiment, the first face and the second face are defined by the first isolating portion similar to the above-described configuration.

In an optional embodiment, the first jaw includes a distal end face, wherein the first electrode, the second electrode, and/or the first isolating portion are exposed on the distal end face.

The first and second electrodes can operate to provide a localised seal for and/or cut in a biological vessel/tissue gripped between the first and second jaws. Further, the first electrode and the second electrode can be further used for radiofrequency cutting at the distal end face. In a position of the first and second jaws in which the second jaw does not cover the section of the first electrode and the second electrode exposed on the distal end face (e.g. the open position), the sections of the first electrode and the second electrode that are exposed on the distal end face can act as an active electrode and a return electrode for radiofrequency cutting. This may allow fine cutting at the distal end face of the electrosurgical instrument, for example, for cutting a hole into tissue so that the electrosurgical instrument can be further advanced into and/or through the tissue, for example, as part of a tunnelling procedure . Further, the first and second electrodes exposed on the distal end face can be used to cut fine and/or small sections of tissue that are clamped or grasped between the distal end face and the second jaw. Thus, tissue can be "nibbled".

The first surface includes the first electrode and some exposed sections of the second electrode. Other sections of the first electrode and the second electrode are exposed on the distal end face. The first electrode and/or the second electrode may be connected to an inner conductor and an outer conductor of the coaxial cable, respectively.

The sections of first electrode and the second electrode that are exposed on the distal end face are spaced apart from each other, for example by an air gap or the first isolating portion (e.g. an exposed section thereof).

The first surface and the distal end face may form a continuous surface of the first jaw. The first surface and the distal end face may be inclined relative to each other. For example, an angle between a plane defined by the first surface and a plane defined by the distal end face may form an angle between 10° to 90°, optionally 45°, 60°, or 90°. The distal end face, the first surface, and an outer surface of the second electrode may define an outer surface of the first jaw. In this configuration, the distal end face is a side surface while the first surface and the outer surface of the second electrode are surface that extends along the longitudinal direction of the first jaw. In other words, the distal end face extends to transverse to the longitudinal direction of the first jaw. The distal end face may be a surface of the first jaw that is arranged at a distal-most position of the first jaw. In other words, when moving the first jaw along the longitudinal direction of the first jaw towards the tissue, the distal end face firstly and/or solely contacts the tissue.

The distal end face may be straight/flat. Alternatively, the distal end face may be curved. The sections of the first electrode, the second electrode, and the first isolating portion that are exposed at the distal end face may be flush with respect to each other or define a flat surface. Alternatively, the sections of the first electrode and/or the second electrode that are exposed at the distal end face may protrude from the section of the first isolating portion that is exposed at the distal end face.

The second jaw (e.g. the third electrode) may partially or completely cover the first surface and/or a distal end face in the closed position of the first jaw and the second jaw. In any case, the second jaw (e.g. the third electrode) covers the section of the first electrode that is exposed at the distal end face and/or the section of the second electrode that is exposed to the distal end face in a closed position. Stated differently, if the first jaw and the second jaw are brought together with no tissue therebetween, the second jaw covers and/or contacts the sections of the first electrode and/or the second electrode that are exposed on the first surface and/or the distal end face. In this way, the second jaw may function to shield the first and/or second electrode(s) when the jaws are closed, for example, to avoid unintentionally treating tissue whilst the instrument is moved into position at a treatment site.

The sections of the first electrode and the second electrode exposed at a distal end face can define an active electrode and a return electrode, respectively, which can be used for cutting tissue at the distal end face. The first electrode and the second electrode can be connected to the transmission line which is configured to convey both microwave and radiofrequency energy.

In an optional embodiment, the second jaw includes an overhang portion protruding from the second surface. Optionally the overhang portion covers sections of the first electrode and/or the second electrode that are exposed on the distal end face in the closed position of the first jaw and the second jaw.

The overhang portion may be provided by the third electrode and may include a side surface which is continuous with the second surface. The second surface and the side surface may be inclined to each other. For example, an angle between a plane defined by the second surface and a plane defined by the side surface may form an angle between 10° to 90°, optionally 45°, 60°, or 90°. This angle may correspond to (e.g. be identical to) the angle defined by the first surface and the distal end face. In the closed position, the distal end face and side surface may extend parallel to each other and/or contact each other.

The overhang portion of the second jaw may provide the first and second jaws with a shape similar to a hooked-shaped beak of a bird of prey. In the longitudinal direction of the first jaw and the second jaw (e.g. measured from the pivot axle), the second jaw may have a greater length along the longitudinal direction compared to the first jaw in the closed position. The difference in length between the first jaw and the second jaw can correspond to the length of the overhang portion along the longitudinal direction. In other words, without the overhang portion, the first jaw and the second jaw may have the same length and/or configuration. The overhang portion protrudes from the second surface towards the first jaw and partially or completely covers the distal end face.

Tissue can be clamped or grasped between the distal end face and the side surface of the overhang portion. The surface areas of the side surface and the distal end face are significantly smaller than the surface areas of the first surface and the second surface. Therefore, smaller portions of tissue can be grasped between the distal end face and the side surface compared to clamping tissue between first surface and the second surface. This allows finer cutting of tissue that is clamped between the distal end face and the side surface.

In an optional embodiment, the electrosurgical instrument further comprises a joint having a pivot axis and an arm movable relative to the second jaw (and the first jaw).

As the arm is movable relative to the second jaw, pressure that is applied by the arm on the tissue which is clamped or grasped between the first jaw and the second jaw in the closed position can be varied relative to the pressure that is applied by the first jaw and the second jaw (i.e. by the first surface and the second surface). This means that different portions of the tissue can be subjected to different pressures. For example, tissue that is clamped between the first surface and the second surface may be subjected to a constant pressure (e.g. for microwave sealing, such as during the emission of microwave energy) while tissue that is arranged between the arm and the first surface may be subjected to a different pressure. For example, the pressure applied to this part of the tissue may be increased by moving the arm towards or to the press position or reduced by moving the arm towards or to the relaxed position. For example, during the emission of microwave energy, the first jaw and the second jaw are in the closed position and the arm is in the relaxed position. During the emission of radiofrequency energy, the arm may be in the press position for increasing the pressure on the area subjected to radiofrequency cutting while the first jaw and the second jaw either remain in the closed position or are slightly opened compared to the closed position so that tissue that has been cut is released from the electrosurgical instrument. In an alternative use case, the same instrument may be controlled to provide relatively more pressure for microwave sealing and relatively less pressure for radiofrequency cutting. The invention provides a mechanism for varying the pressure during microwave sealing and radiofrequency cutting.

The press position is a position of the arm in which the arm is configured to press against tissue that is clamped or grasped between the first jaw and the second jaw in the closed position. For example, a contact surface of the arm (e.g. an outer surface of the arm that is in contact with the tissue in the press position) can apply more pressure on the tissue compared to the pressures applied by the first jaw and the second jaw (i.e. between the first surface and the second surface). For example, the contact surface of the arm protrudes from the second surface in the press position and/or is closer to the first surface compared to the second surface in the press position. An angle defined between the first surface and the second surface may be greater than an angle defined between the contact surface of the arm and the first surface in the press position.

In the relaxed position, the contact surface of the arm may be arranged further away from the first surface compared to the second surface (e.g. in the closed position). So, the arm applies less pressure to the tissue in the relaxed position compared to the pressure that is applied between the first jaw and a second jaw in the closed position. An angle defined between the first surface and the second surface may be smaller than an angle defined between the contact surface of the arm and the first surface in the relaxed position.

The arm may be actuated by a further control wire or actuation rod. There may be a further actuation mechanism (e.g. implemented with the joint) that converts a back-and-forth movement of the actuation rod or control wire for the arm into a rotational movement of the arm. In the press position, the arm may be (continuously) actuated to apply a (constant) pressure on the tissue. In the relaxed position, the arm may not be actuated so that the arm rests on the tissue (e.g. due to gravity) or (freely) moves away from the tissue depending on the orientation of the electrosurgical instrument. Alternatively, the arm may be actuated away from the first jaw for providing the relaxed position. In this case, the arm may be positioned so that the contact surface does not contact the tissue that is clamped between the first jaw and the second jaw.

In an optional embodiment, the second jaw is rotatable around a pivot axis of the joint. Optionally, the arm is also rotatable around the pivot axis. Further optionally, the second jaw and the arm are configured to be rotated independently from each other.

The arm may also be attached to the joint. For example, the arm may be pivotable around the pivot axis. The arm may be freely movable with respect to the second jaw. Stated differently, the arm is not (mechanically) coupled to the second jaw so that a movement of the second jaw does not result in a movement of the arm and vice versa. In other words, the second jaw and the arm may be actuated completely separately from each other. For example, the arm may only be rotatably attached to the pivot axle. Further, the second jaw is only rotatably attached to the pivot axle. So, the arm and the second jaw can rotate independently from each other around the pivot axis.

The rotation around the same pivot axis and/or the attachment of the arm and the second jaw to the same pivot axle provides that the contact surface of the arm and the second surface of the second jaw can form a common and/or flush surface along the extension of the second jaw irrespective of the position of the second jaw or the arm as long as both elements are arranged at the same position/angle. The first jaw may also be pivotally coupled to the pivot axle. For example, the joint includes a pivot axle to which the first jaw, the second jaw, and/or the arm is rotatably attached. The joint may rotatably support the pivot axle.

In an optional embodiment, the second jaw includes a jaw slot and the arm includes an arm cam, wherein the arm cam is inserted in the jaw slot for providing movement of the arm relative to the second jaw between the retracted position and the press position.

The jaw slot and the arm cam may be provided in addition to the rotatable attachment of the arm to the pivot axle. The arm may include one or more arm cams and/or the second jaw includes one or more jaw slot. Optionally, each arm cam is inserted in a respective jaw slot.

The jaw slot is an elongated slot having a width slightly greater than a diameter of the arm cam and a length significantly greater than the diameter of the arm cam so that the arm cam can slide within the jaw slot along the length of the jaw slot. A movement of the arm cam in the jaw slot corresponds to a relative movement of the arm with respect to the second jaw. If the arm cam abuts against a first end of the jaw slot, the arm can be in the press position. If the arm cam abuts against a second opposite end of the jaw slot, the arm can be in the relaxed position. So, the length of the jaw slot determines the angular range or movement range within which the arm can move relative to the second jaw.

In this embodiment, the arm is coupled to the second jaw so that a movement of the second jaw results in a movement of the arm. The actuation of the arm results in a movement of the arm relative to the second jaw. This configuration may be helpful for moving the arm relative to the second jaw, i.e. between the press position and the relaxed position, since the actuation of the arm is relative to the position of the second jaw.

In conclusion, the arm may also be attached to the joint. In an embodiment, the arm may be pivotable around the pivot axis. The arm may be moved independently from the second jaw. In an alternative embodiment, the arm may be pivotable with respect to the second jaw, i.e. is pivotally attached to the second jaw. In this latter arrangement, movement of the second jaw also moves the arm so that the arm can be moved relative to the second jaw.

In an optional embodiment, the second jaw and/or the second surface include an opening through which the arm can be moved.

The opening of the second jaw may completely extend through the second jaw so that the arm can be moved from one side of the second jaw, through the opening, and to the other side of the second jaw. The opening may be a through-hole which extends from the second surface to the other side of the second jaw.

The outer shape of the arm may match the shape of the opening. There may be a small gap between the outer perimeter of the arm and the inner surface of the opening. Alternatively, the inner surface of the opening provides a sliding contact with the arm.

The second jaw may have a recess within which the arm can be received. In this case, the opening corresponds to the open portion of the recess. The arm may thus be movable through the second surface between the retracted position and the press position. For example, the arm abuts against a bottom (e.g. innermost) surface of the recess in the relaxed position and protrudes from the recess in the press position.

Similarly, in case of a through-hole in the second jaw, the arm protrudes from the opening in the second jaw in the press position. In the retracted position, the arm can protrude from an outer surface of the second jaw (e.g. back surface of the second jaw).

In an optional embodiment, the arm includes a second isolating portion which is configured to be in contact with the first electrode in the press position of the arm (in the absence of tissue between the first jaw and the second jaw and/or in the closed position).

In this embodiment, tissue can be sealed using microwave energy in the closed position whereby the arm can be positioned away from the first surface (e.g. the retracted position) - for example the arm is arranged outside of the opening. In this case, little or no pressure is applied on tissue over the area of the gap (i.e. between the first sealing area the second sealing area) and/or over the first/radiofrequency electrode arranged on the second isolating portion. For cutting the tissue, the arm is then moved through the opening so that the arm presses the tissue against the third electrode arranged on the first jaw (the press position). So the pressure on the tissue can be increased for radiofrequency cutting. So, differing levels of pressure may be applied during sealing and cutting. For example, a higher pressure may improve a seal, but a lower pressure may improve a cut.

The second isolating portion may define the contact surface of the arm that faces the radiofrequency/first electrode. Optionally, the second isolating portion contacts the first electrode and/or extends parallel to the first electrode in the press position (in the absence of tissue). The second isolating portion arranged on the arm may be made from the same electrically non-conductive material as the first isolating portion. The electrically conductive part of the arm may support the third isolation portion.

In an optional embodiment, the arm and the second jaw respectively include conductive materials which form the third electrode.

The conductive material of the second jaw may have the shape of a half-shell except for the opening in which the conductive material of the arm can be arranged if the arm is appropriately positioned relative to the second jaw. In this position, the conductive materials of the arm and the second jaw may form a continuous half-shell except for a (small) gap between the arm and a perimeter of the opening.

In an optional embodiment, the second jaw includes a third isolating portion which covers the opening and is flexible across the opening so that the arm is configured to deform the third isolating portion in the closed position or the arm deforms the third isolating portion in the press position of the arm, in which the arm and the first surface are brought together to cut tissue therebetween, wherein optionally the third isolating portion is made from silicon.

In this embodiment, the third isolating portion may cover the opening. The section of the third isolating portion that covers the opening may be thin and/or have the configuration of a membrane across the opening. This provides sufficient flexibility and/or softness of the third isolating portion over the opening so that the third isolating portion can be deformed. For example, the third isolating portion includes a membrane section providing a membrane that covers the opening. The membrane section may be unitary component with the rest of the third isolating portion.

The membrane section or the complete third isolating portion may be made from silicon or a silicone-based material which provides the elasticity of the membrane section.

The provision of the third isolating portion that covers the opening reduces that risk that tissue is pinched between the arm and the second jaw.

In an optional embodiment, the first isolating portion includes a first material portion and a second material portion. Optionally, the first material portion is arranged between the transmission line and the second material portion, and wherein a material of the first material portion is different to a material of the second material portion.

The first material portion and a second material portion may completely surround the first electrode except for the part where the second electrode protrudes from the first isolating portion. The first material portion may be in contact with the transmission line. For example, the first material portion is in contact with the dielectric material of the coaxial cable of transmission. The second material portion may not be in contact with the transmission line. The first material portion is arranged between the second material portion and the transmission line. For example, the first material portion may not be exposed at the first surface - only the second material portion is exposed at the first surface.

Optionally, the second material portion includes silicon or silicon-based material. The first material portion may include silicone and/or Aluminia and/or Macor ceramic. The first material portion may be considered a launch section. In a simplified explanation, the first material portion acts as a transformer to match a microwave antenna provided by the first electrode and the second electrode. Essentially, changing the dielectric material changes the dielectric constant which in turn changes the impedance of that section. Then the transformer may need to have the correct length (typically ¼ wavelength or odd multiple) to match at the specified frequency. There is also possibility to use a half wavelength.

According to a second aspect of the present disclosure, there is provided an electrosurgical instrument for sealing and/or cutting tissue, which comprises an instrument shaft comprising a transmission line for conveying microwave electromagnetic energy, a first jaw attached to the instrument shaft and including a first surface, a second jaw attached to the instrument shaft and including a second surface, a first electrode for emitting microwave and/or radiofrequency electromagnetic energy, a second electrode for emitting microwave and/or radiofrequency electromagnetic energy, a first isolating portion, and a second isolating portion, wherein the first jaw and the second jaw can be moved between an open position, in which the tissue can be inserted between the first surface and the second surface, and a closed position, in which the first and second surfaces are brought together to clamp the tissue therebetween, wherein the first electrode is the sole electrode arranged on the first jaw and the second electrode is arranged on the second jaw, wherein the first isolating portion electrically isolates the first electrode from the second electrode in the closed position, and wherein the first isolating portion covers the first electrode except for a ridge element that is exposed on the first surface and configured to contact the second isolating portion in the closed position.

The above comments, features, and/or optional embodiments relating to the transmission line, the instrument shaft, the first jaw, the second jaw, the first electrode, and/or the first isolating portion equally apply to the second aspect of the electrosurgical instrument. Further, the comments, features, and/or optional embodiments on the second aspect equally apply to the first aspect if applicable.

This electrosurgical instrument may be used for microwave sealing and/or radiofrequency cutting. However, these functions cannot be provided by the first jaw alone as with the previous embodiments. Microwave sealing and/or radiofrequency cutting occurs between the first jaw and the second jaw, i.e. between the first electrode on the first jaw and the second electrode on the second jaw.

The electrosurgical instrument may have the same features of the electrosurgical instrument of the first aspect but there is no second electrode. The second electrode of the second aspect corresponds to the third electrode of the first aspect.

The first isolating material may cover the first electrode except for the exposed section or a ridge of the first electrode. Microwave sealing and/or radiofrequency cutting are applied between the first electrode and the second electrode on the second jaw.

According to a third aspect of the present disclosure, there is provided an electrosurgical instrument for sealing and/or cutting tissue, comprising an instrument shaft comprising a transmission line for conveying microwave and/or radiofrequency electromagnetic energy; a first jaw attached to the instrument shaft and including a first surface, a second jaw attached to the instrument shaft and including a second surface, a first electrode for emitting microwave and radiofrequency energy, a second electrode for emitting microwave and/or radiofrequency energy, a third electrode for emitting microwave and/or radiofrequency energy, and a first isolating portion electrically isolating the first electrode from the second electrode, wherein the first jaw and the second jaw can be moved between an open position, in which the tissue can be inserted between the first surface and the second surface, and a closed position, in which the first and second surfaces are brought together to clamp the tissue therebetween, wherein the first electrode and the second electrode are arranged on the first jaw, wherein the first electrode is exposed on the first isolating portion, wherein the third electrode is arranged on the second jaw, wherein the third electrode forms a third face and a fourth face, and wherein the third face and the fourth face contact the first isolating portion in the closed position.

The above comments, features, and/or optional embodiments relating to the transmission line, the instrument shaft, the first jaw, the second jaw, the first electrode, the second electrode, the third electrode, the first isolating portion and/or the second isolating portion equally apply to the third aspect of the electrosurgical instrument. Further, the comments, features, and/or optional embodiments on the first aspect equally apply to the third aspect if applicable. The difference of the third aspect to the first aspect is that the first electrode of the third aspect can be only exposed (e.g. flush with the first isolating portion) but not necessarily protrudes from the first isolating portion.

According to a fourth aspect of the present disclosure, there is provided an electrosurgical apparatus for sealing and cutting tissue which comprises a generator unit for generating radiofrequency and/or microwave electromagnetic energy and the electromagnetic electrosurgical instrument as described above. The transmission line is configured to convey the radiofrequency and/or microwave electromagnetic energy from the generator unit to first electrode, the second electrode and/or the third electrode.

The generator unit may be configured to generate electromagnetic energy of a fixed single frequency or of a plurality of fixed single frequencies. Alternatively or additionally, the generator unit may be tuneable to generate electromagnetic energy of various frequencies, for example in a continuous range of frequencies between a minimum frequency and a maximum frequency. The generator unit may be connected to a power supply which provides the energy for generating the radiofrequency electromagnetic energy and/or microwave electromagnetic energy.

The generator unit is electrically and/or electronically (directly or indirectly) connected to the transmission line. Optionally, the generator unit generates the radiofrequency energy and/or microwave energy which is conveyed by the transmission line to the first to third electrodes where the radiofrequency energy and/or microwave energy is radiated into the treatment zone.

In an optional embodiment, the generator unit is configured to simultaneously generate microwave electromagnetic energy of the first frequency and microwave electromagnetic energy of a second frequency.

For example, the generator unit includes a generator that is configured to simultaneously generate electromagnetic energy of two different (fixed) frequencies.

Alternatively, the generator unit includes a first generator for generating electromagnetic energy of the first frequency and a second generator for generating electromagnetic energy of the second frequency. The output of the first generator and output of the second generator can be combined using a multiplexer.

The multiplexer may be a diplexer and can combine the input from various sources into one output. For example, a multiplexer (or diplexer) is used to combine the output of first and second generators to a single output which is connected or coupled to the transmission line.

In an optional embodiment, the generator unit is configured to simultaneously or alternatingly generate microwave electromagnetic energy of the first frequency and radiofrequency electromagnetic energy of a third frequency.

The generator unit may include a first generator for generating electromagnetic energy of the first frequency, a second generator for generating electromagnetic energy of the second frequency, and/or a third generator for generating electromagnetic energy of the third frequency. The output of the first generator and the output of the second generator may be combined as described above using a multiplexer.

The output of the third generator may be combined with the output of the multiplexer using a combiner which can include a switch for alternatingly switching between outputting the output of the multiplexer and outputting the output of the third generator. The combiner may include an additional multiplexer for combining the output of the multiplexer and the output of the third generator to simultaneously emit electromagnetic energy of the first frequency, the second frequency, and the third frequency. In this case, microwave sealing and radiofrequency cutting can be simultaneously effected.

If switching between the output of microwave energy and radiofrequency energy is possible, this can be used for sealing the tissue using microwave energy and then subsequently cutting the tissue using radiofrequency energy. Alternatively, the switch between the output of microwave energy and radiofrequency energy can be executed repeatedly and rapidly providing near simultaneous cutting and sealing.

### BRIEF DESCRIPTION OF THE DRAWINGS

Embodiments of the disclosure are described in detail below with reference to the accompanying drawings, in which:
Fig. 1 shows a schematic view of an embodiment of an electrosurgical apparatus;
Figs. 2a and 2b show perspective views of a further embodiment of an electrosurgical instrument (in an open position) of the electrosurgical apparatus shown in Fig. 1 in different positions of a second jaw and an arm,
Fig. 3 shows a schematic cross-sectional view of a further embodiment of an electrosurgical instrument (in an open position) of the electrosurgical apparatus shown in Fig. 1;
Fig. 4 shows a schematic cross-sectional view of a further embodiment of an electrosurgical instrument (in an open position) of the electrosurgical apparatus shown in Fig. 1;
Fig. 5 shows a schematic cross-sectional view of a further embodiment of an electrosurgical instrument (in an open position) of the electrosurgical apparatus shown in Fig. 1;
Fig. 6 shows a schematic cross-sectional view of a further embodiment of an electrosurgical instrument (in an open position) of the electrosurgical apparatus shown in Fig. 1;
Fig. 7 shows a perspective view of a further embodiment of an electrosurgical instrument (in an open position) of the electrosurgical apparatus shown in Fig. 1;
Fig. 8 shows a side-view of the electrosurgical instrument Fig. 7;
Fig. 9 shows a side-view of the electrosurgical instrument Fig. 7 with a first isolating portion removed;
Fig. 10 shows a perspective view of a further embodiment of an electrosurgical instrument (in an intermediate position) of the electrosurgical apparatus shown in Fig. 1, whereby a lumen is empty;
Fig. 11 shows a perspective of the electrosurgical instrument Fig. 10 with a blocking element arranged in the lumen;
Fig. 12 shows a perspective view of a further embodiment of an electrosurgical instrument (in an open position) of the electrosurgical apparatus shown in Fig. 1;
Fig. 13 shows a cross-sectional perspective view of the electrosurgical instrument Fig. 12;
Fig. 14 shows a schematic perspective view of a further embodiment of an electrosurgical instrument (in closed position) of the electrosurgical apparatus shown in Fig. 1;
Fig. 15 shows a cross-sectional view of the electrosurgical instrument Fig. 14;
Fig. 16 shows a schematic perspective view of a further embodiment of an electrosurgical instrument (in closed position) of the electrosurgical apparatus shown in Fig. 1;
Fig. 17 shows a schematic perspective view of a further embodiment of an electrosurgical instrument (in closed position) of the electrosurgical apparatus shown in Fig. 1.
Fig. 18 shows a schematic cross-sectional view of a further embodiment of an electrosurgical instrument (in closed position) of the electrosurgical apparatus shown in Fig. 1;
Fig. 19 shows a cross-sectional view of the electrosurgical instrument Fig. 12, wherein a second jaw is further moved towards a first surface relative to the closed position so that a recessed portion is moved closer towards a first electrode;
Fig. 20 shows a perspective view of a further embodiment of an electrosurgical instrument (in an open position) of the electrosurgical apparatus shown in Fig. 1, wherein a channel and a second isolating portion are highlighted;
Figs. 21a and 21b show enlarged perspective views of the electrosurgical instrument of Fig. 20 in a first orientation of a blocking element (Fig. 21a) and a second orientation of the blocking element (Fig. 21b), wherein the channel and the second isolating portion are highlighted; and
Figs. 22a and 22b show enlarged perspective views of a further embodiment of an electrosurgical instrument (in a closed position) of the electrosurgical apparatus shown in Fig. 1 in the absence of a blocking element (Fig. 22a) and in the presence of the blocking element (Fig. 22b), wherein a channel and a second isolating portion are highlighted.

### DETAILED DESCRIPTION; FURTHER OPTIONS AND PREFERENCES

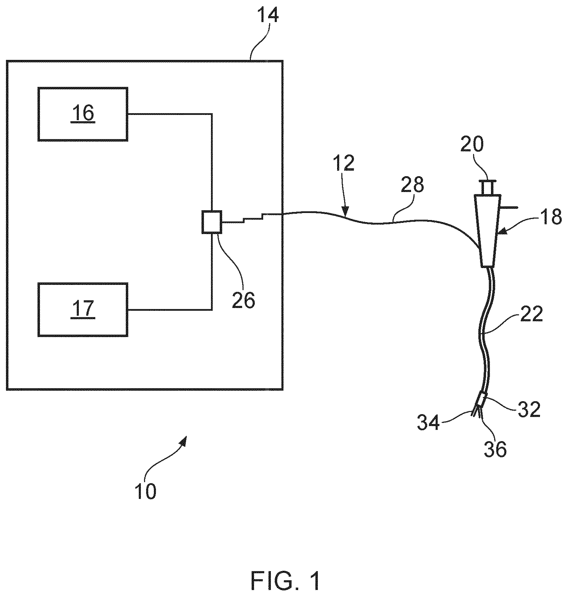

The present disclosure relates to an electrosurgical instrument and apparatus capable of delivering microwave energy to seal tissues (e.g. blood vessels) and of cutting the tissue. The electrosurgical instrument and apparatus may be used in open surgery but may find particular use in procedures where there is restricted access to the treatment site. For example, the electrosurgical instrument of the disclosure may be adapted to fit within the instrument channel of a surgical scoping device i.e. laparoscope, endoscope, or the like. Fig. 1 shows a schematic view of an electrosurgical apparatus 10 in which the electrosurgical instrument of the disclosure may be used.

Fig. 1 is a schematic diagram of a complete electrosurgical apparatus 10 that is an embodiment of the invention. The electrosurgical apparatus 10 is arranged to treat biological tissue using radiofrequency (RF) and/or microwave electromagnetic (EM) energy delivered from an electrosurgical instrument 12. The electromagnetic energy emitted by the electrosurgical instrument 12 into a treatment zone can be used to coagulate, cut, and/or ablate tissue in the treatment zone.

The electrosurgical apparatus 10 further comprises a generator unit 14 which can controllably supply radiofrequency and/or microwave electromagnetic energy to the electrosurgical instrument 12. The generator unit 14 may include a first generator 16 and a second generator 17. Suitable generators for this purpose are described in WO 2012/076844. The generator unit 14 may be arranged to monitor reflected signals received back from the electrosurgical instrument 12 in order to determine an appropriate power level for delivery. For example, the generator unit 14 may be arranged to calculate an impedance seen at the electrosurgical instrument 12 in order to determine an optimal delivery power level.

The electrosurgical apparatus 10 further comprises a surgical scoping device 18, such as a bronchoscope, endoscope, gastroscope, laparoscope or the like. The scoping device 18 may include a handpiece 20 and a flexible shaft 22. The handpiece 20 may include means for guiding the flexible shaft 22 through a cavity of a body. For example, the handpiece 20 can include means for moving a distal end of the flexible shaft 22 to change direction of the distal end of the flexible shaft 22. This helps manoeuvring the flexible shaft 22 through the cavity of the body. The flexible shaft 22 may include a working channel through which elongated structures can be moved and, thus, positioned at the treatment zone within the cavity of the body.

The first generator 16 and the second generator 17 are each configured to generate electromagnetic energy of a fixed frequency. However, the generator unit 14 is not limited thereto; the first generator 16 and/or the second generator 17 can be configured to generate AC electromagnetic energy in a continuous range between a minimum frequency and a maximum frequency. The frequency of the electromagnetic energy to be generated by the first generator 16 and/or the second generator 17 may be selected using an interface (not shown in the figures).

The generator unit 14 can include a combiner 26 which is configured to temporally switch between outputting the output of the first generator 16 or the output of the second generator 17. The combiner 26 may also be configured to combine the outputs of the first generator 16 and of the second generator 17. In this case, the combiner 26 acts as a multiplexer or diplexer.

The generator unit 14 is thus capable of generating and controlling power to be delivered to the electrosurgical instrument 12, e.g. via a transmission line, which extends from the generator unit 14 through the surgical scoping device 18 and instrument channel to the distal tip of the instrument channel. The generator unit 14 may have a user interface for selecting and/or controlling the power delivered to the electrosurgical instrument 12, e.g. controlling the first and/or the second generators 16, 18 and/or the combiner 26. The generator unit 14 may have a display for showing the selected energy delivery mode. In some examples, the generator unit 14 may allow for an energy delivery mode to be selected based on the size of the vessel to be sealed.

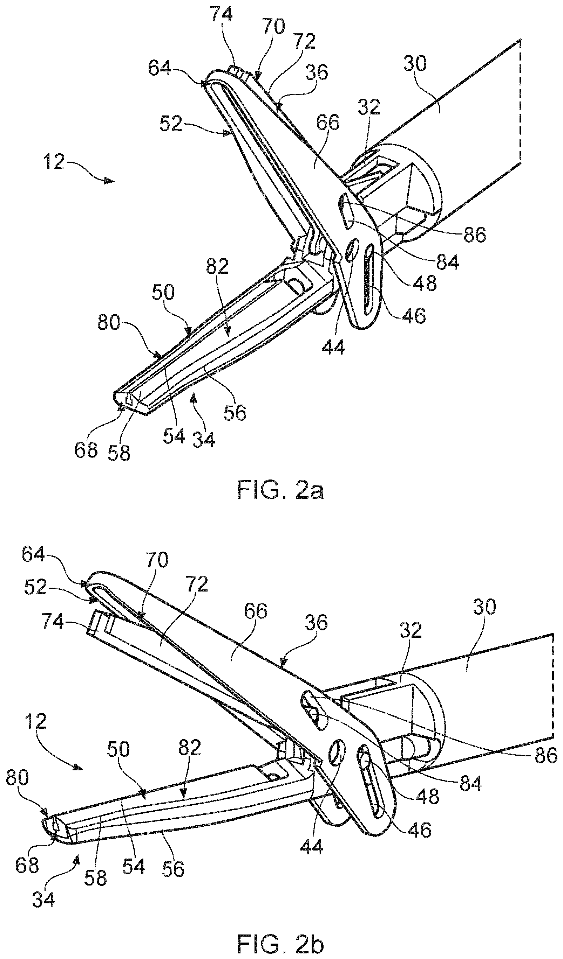

As exemplarily shown in Figs. 2a and 2b, the electrosurgical instrument 12 can include the transmission line, an instrument shaft 30, a joint 32, a first jaw 34, a second jaw 36, and/or an arm 70 which will be discussed in further detail below. The transmission line may include a single coaxial cable that connects the generator unit 14 to the first jaw 34 and/or second jaw 36 for conveying the radiofrequency and/or microwave energy.

Figs. 2a and 2b show a schematic perspective view of a distal end of an embodiment of the electrosurgical instrument 12. The second jaw 36 is rotatably or pivotally connected or coupled to the instrument shaft 30 which is dimensioned to fit within the instrument channel of the surgical scoping device 18. The first jaw 24 is fixed relative to the instrument shaft 30, i.e. not movable or actuatable. The instrument shaft 30 comprises a tubular sheath that covers the transmission line for carrying microwave and/or radiofrequency energy to the jaws 34, 36 together with various control wires or (actuation) rods that are arranged to control and/or physically manipulate the first and second jaws 34, 36, as discussed below.

The second jaw 36 is operably coupled to the joint 32 that is mounted on a distal end of the instrument shaft 30. So, the pair of jaws 34, 36 comprises a static jaw (the first jaw 34) that is fixed relative to the instrument shaft 30 or the joint 32. The other jaw (second jaw 36) is pivotable or rotatable.

In an alternative embodiment, both the first and second jaws 34, 36 may be arranged to pivot relative to the joint 32. The joint 32 may be arranged to ensure that the jaws remain laterally aligned as they are moved together.

In the embodiment shown, the joint 32 includes a pivot axle 44 which defines a pivot axis. The second jaw 36 can pivot around the pivot axis or pivot axle 44. For example, the pivot axle 44 is fixed to the joint 32 and the second jaw 36 can rotate around the pivot axle 44.

The joint may have a clevis structure. The second jaw 36 may include elongated slots 46 (see Figs. 2a and 2b). A control wire or actuation rod includes a cam 48 which is inserted in the slots 46 of the second jaw 36. The engagement of the cam 48 with the slots 46 provides an actuation mechanism which translates a back-and-forth movement of the control wire (and thus of the cam 48) into a rotational movement of the first jaw 34 and the second jaw 36 around the pivot axle 44.

In use, the first jaw 34 and the second jaw 36 are intended to grip biological tissue (in particular a blood vessel) therebetween. The first jaw 34 and the second jaw 36 are arranged to apply pressure to the biological tissue between the opposed surfaces of the jaws 34, 36 and deliver energy (preferably microwave and/or radiofrequency electromagnetic energy) into the tissue from the transmission line.

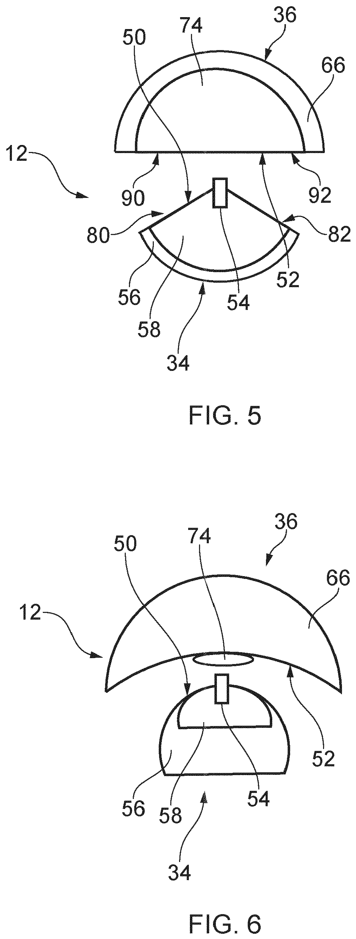

The first jaw 34 includes a first surface 50 which opposes a second surface 52 of the second jaw 36. The first surface 50 and/or the second surface 52 may form an outer surface of the first jaw 34 and the second jaw 36 respectively, which can be brought into contact with each other when the jaws 34, 36 are in the closed position (see Fig. 5). For example, the first surface 50 and the second surface 52 may be considered pressure pads or pressure areas with which pressure can be applied to the tissue grasped between the first jaw 34 and the second jaw 36.

In the embodiment of Figs. 2a and 2b, the first jaw 34 includes a first electrode 54, a second electrode 56, and/or a first isolating portion 58. The first electrode 54 and the second electrode 56 are made from an electrically conductive material, such as metal or a metal alloy. The second electrode 56 may form the outer surface of the first jaw 34 and/or may provide the connection to the instrument shaft 30. Thus, the second electrode 56 may have a function of providing the stability of the first jaw 34.

The second electrode 56 may have a form of a half-shell in a portion along the first surface 50. The second electrode 56 may surround the first electrode 54 and the first isolating portion 58. This means that the first electrode 54 and the first isolating portion 58 may be embedded in the half-shell of the second electrode 56. The shape of the second electrode 56 may also be considered as providing a recess or channel in which the first electrode 54 and the first isolating portion 58 are arranged.

The first isolating portion 58 electrically isolates the first electrode 54 from the second electrode 56. The first isolating portion 58 may be made from an electrically non-conductive material such as ceramics (e.g. including Zirconia), PEEK, silicon, and/or other plastic materials. The first isolating portion 58 may be sandwiched between the first electrode 56 and the second electrode 58.

The second electrode 58 may have a U-shape or V-shape in a cross-sectional view along a section of the first jaw 34. Further, the second electrode 58 may have a plate-shape (along this section of the first jaw 34. At a distal end and/or a proximal end of the first jaw 34, the second electrode 58 may have a different configuration so that the second electrode 58 does not have an open end but a closed end (not shown in the figures). This means that the second electrode 56 can shield the first electrode 54 in all directions away from the first surface 50.

The first electrode 54 and/or the second electrode 56 are exposed at the first surface 50 for getting in contact with the tissue clamped between the first jaw 34 and the second jaw 36. In particular, two sections of the second electrode 56 are exposed on the first surface 50 which are spaced from each other by a gap. In the embodiment shown, the two exposed sections are straight and extend in a direction of extension of the first jaw 34.

The first electrode 54 is configured to emit microwave and radiofrequency energy for tissue sealing and cutting, respectively. The first electrode 54 is electrically isolated from the second electrode 56 and/or a third electrode 66. The first electrode 54 may be an active electrode for delivering radiofrequency electromagnetic energy while the second electrode 56 and/or the third electrode 66 are return electrodes. The first electrode 54 is connected to the transmission line, optionally to the coaxial cable.

The first electrode 54 is exposed on the first isolating portion 58 and/or protrudes from the first isolating portion 58. The first electrode 54 is arranged to first contact tissue that is clamped between the first jaw 34 and the second jaw 36. In other words, the first electrode 54 protrudes from the first isolating portion 58 in a direction towards the second jaw 36. The first electrode 54 may be a ridge or bar made from an electrically conductive material, such a metal or a metal alloy. The first isolating portion 58 is made from an electrically non-conductive material, such as a ceramic or plastic material.

The first electrode 54 is arranged in the middle between the two sections of the second electrode 56 that are exposed on the first surface 50. The exposed sections of the second electrode 56 and of the first isolating portion 58 may be symmetrical to the first electrode 54. The first electrode 54 may divide the first surface 50 into a first face 80 and a second face 82. The first face 80 and the second face 82 may be symmetrical to the first electrode 54. The first face 80 as well as the second face 82 may each include an exposed section of the second electrode 56 and/or an exposed section of the first isolating portion 58.

The first face 80 and the second face 82 define an angle of less than 180° with respect to each other. As the first electrode 54 is arranged between the first face 80 and the second face 82, the protruding effect of the first electrode 54 (of increasing pressure on the tissue) is further increased.

The first electrode 54 and the second electrode 56 are configured to emit microwave electromagnetic energy. Further, the first electrode 54 is configured to emit radiofrequency energy. For example, the first electrode 54 acts as an active electrode while the second electrode 56 and/or the third electrode 66 act as return electrodes.

The first jaw 34 further includes a distal end face 68 which is directly adjacent to the first surface 50. The distal end face 68 is inclined to the first surface 50 by an angle of 90°. The distal end face 68 is a side surface of the first jaw 34 that firstly comes in contact with tissue if the first jaw 34 is advanced in the longitudinal direction of the first jaw 34 towards the tissue. In the embodiment of Figs. 2a and 2b, the distal end face 68 is flat. The first electrode 54, the second electrode 56, and/or the first isolating portion 58 are exposed on the distal end face 68. The sections of the second electrode 56 that are exposed on the distal end face 68 are spaced from each other. Further, the section of the first electrode that is exposed on the distal end face 68 is arranged between the sections of the second electrode 56 that are exposed on the distal end face 68. The sections of the first electrode 54 and the second electrode 56 that are exposed on the distal end face 68 are continuous with the respective sections of the first electrode 54 and the second electrode 56 that exposed on the first surface 50. As such, the sections of the first electrode 54 and the second electrode 56 that exposed on the distal end face 68 provide one active electrode and two return electrodes for radiofrequency cutting. So, the first electrode 54 and the second electrode 56 are connected to the transmission line which is configured to convey both microwave energy and radiofrequency energy e.g. includes a coaxial cable or a coaxial cable for conveying microwave energy and wires for conveying radiofrequency energy.

The surface area of the distal end face 68 is significantly smaller than the surface area of the first surface 50. Similarly, the sections of the first electrode 54 and the second electrode 56 that are exposed on the distal end face 68 are smaller than the respective sections exposed on the first surface 50. This allows fine cutting using the exposed sections of the first electrode 54 and the second electrode 56 on the distal end face 68. For example, the exposed sections of the first electrode 54 and the second electrode 56 on a distal end face 68 may be used to cut holes into the tissue.

The second jaw 36 includes a third electrode 66 and/or the arm 70. The first electrode 54 protrudes from the first isolating portion 58 towards the second jaw 36. A contact surface of the arm 70 (i.e. the surface of the arm 70 that is in contact with the tissue clamped between the first jaw 34 and the second jaw 36) is provided by a second isolating portion 74. The second isolating portion 74 may be attached or fixed to an arm support 72. In the press position, the second isolating portion 74 may be the only part of the arm 70 that is in contact with the tissue or the first electrode 54. Further, in the press position, the first electrode 54 may extend parallel or is in line with the second isolating portion 74.

The third electrode 66 is made from an electrically conductive material, such as metal. The third electrode 66 may form the outer surface of the second jaw 36 and/or may provide the connection to the pivot axle 44. Further, the slots 46 of the second jaw 36 may be arranged on the third electrode 66. Thus, the third electrode 66 may have a function of providing the stability of the second jaw 36.

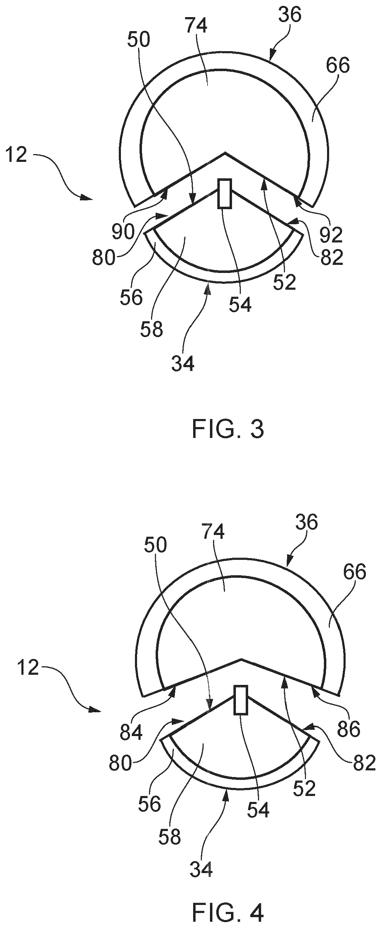

The third electrode 66 may have a form of a half-shell in a portion of the second surface 52. In the embodiments of Figs. 3 to 6, the third electrode 66 may surround the second isolating portion 74. This means that the second isolating portion 74 may be embedded in the half-shell of the third electrode 66. The shape of the third electrode 66 may also be considered as providing a recess or channel in which the second isolating portion 74 arranged.

The third electrode 66 may be mirror-symmetric to the second electrode 56. The third electrode 66 may have a U-shape (see Figs. 3 to 5) in a cross-sectional view along a section of the second jaw 36. Further, the third electrode 66 may have a plate-shape (see Figs. 2 to 5) along this section of the second jaw 36. At a distal end of the second jaw 36, the third electrode 66 may have a different configuration so that third electrode 66 does not have an open end but a closed end. This means that the third electrode 66 can shield the first electrode 54 in all directions away from the second surface 52. The second electrode 56 and the third electrode 66 may be electrically connected to each other, either directly or by being connected to the same conductor of the transmission line.

At a distal end of the second jaw 36, the third electrode 66 may have an overhang portion 64 which includes a side surface or inner surface. The overhang portion 64 may or may not protrude from the second surface 52 towards the first jaw on a distal end of the second jaw 36. However, the overhang portion 64 completely or partially covers the distal end face 68 in the closed position. In particular, the overhang portion 64 covers the sections of the first electrode 54 and the second electrode 56 that are exposed at the distal end face 68. For example, the side surface contacts or provides a small gap with the distal end face 68 in the closed position. As such, tissue can be clamped or grasped between the distal end face 68 and the side surface and/or subsequently cut using radiofrequency energy emitted by the exposed sections of the first electrode 54 and the second electrode 56.

The overhang portion 64 may be a unitary component with the second electrode 66. Optionally, the overhang portion 64 includes an electrically conductive material that is in electrical connection with the second electrode 66. The configuration and arrangement of the second electrode 66 and/or the overhang portion 64 means that third electrode 66 and/or the overhang portion 64 can shield the first electrode 54 in directions away from the second surface 52 and/or the side surface.

The second isolating portion 74 is configured to be in contact with the first electrode 54 in a press position. Fig. 2a shows a retracted position of the arm 70 while Fig. 2b shows an intermediate position between the press position and a retracted position of the arm 70.

As shown in the embodiment of Figs. 2a and 2b, the second jaw 36 may solely consist of the third electrode 66. The second jaw 36 does not include any isolating portion. The electrical isolation of the third electrode 66 with regard to the first electrode 54 is achieved by the second isolating portion 74 on the arm 70 and by the lateral offset of the third electrode 66 with regard to the first electrode 54 in the closed position. The first isolating portion 58 again provides the electrical isolation. The second electrode 56 and the third electrode 66 may have the same electrical potential.

As outlined above, the arm 70 may include the arm support 72 and/or the second isolating portion 74. The arm support 72 may provide the stability of the arm 70, can be made from an electrically conductive material, such as metal, and/or may be rotatably connected to the pivot axle 44. Further, a control wire may be coupled to the arm 70, optionally the arm support 72, for rotating the arm 70 relative to the second jaw 36 and/or around the pivot axle 44.

The third electrode 66 may include an opening or through-hole through which the arm 70 can be moved. The dimensions and/or the shape of the opening may match the ones of the arm 70 so that, when the second jaw 36 and the arm 70 are arranged at the same angular position (i.e. the arm 70 is in line with the second jaw 36 or the second surface 52 is flush with the contact surface of the arm 70), there is a small gap or a sliding contact between the outer surface of the arm 70 and the inner surface of the opening. In particular, the arm support 72 and the third electrode 66 may form a continuous half-shell in this position so that the arm support 72 forms a part of the shielding function of the second electrode 66. The arm support 72 and the third electrode 66 may be electrically connected to each other, either directly or by being connected to the same conductor of the transmission line.

Due to the attachment of the arm 70 to the pivot axle 44, the arm 70 may be freely moved relative to the second jaw 36 so that the arm 70 is in a retracted position. This can be used to reduce pressure between the first electrode 54 and the second isolating portion 74 in the closed position.

The second jaw 36 or the third electrode 66 includes a jaw slot 84 and the arm 70 includes an arm cam 86. The arm cam 86 is inserted in the elongated jaw slot 84 so that the arm 70 is not freely movable with respect to the second jaw 36. Rather, the length of the jaw slot 84 defines the angular range over which the arm 70 can move relative to the second jaw 36. For example, as shown in Fig. 2a, the arm cam 86 abuts against a first end of the jaw slot 84 defining the retracted position. This retracted position corresponds to the maximum rotation of the arm 70 away from the first jaw 34 relative to the second jaw 36.

Fig. 2b shows an intermediate position of the arm 70 relative to the second jaw 36 as the arm cam 86 is arranged between the first end of the jaw slot 84 and an opposing second end the jaw slot 84. In this orientation as well as in the press position, the arm 70 protrudes from the second jaw 36 towards the first jaw 34.

As shown in Fig. 2b, the first jaw 34 and the second jaw 36 are moved towards each other for clamping tissue therebetween (not shown in the figures), i.e. Fig. 2a shows the open position of the first jaw 34 and the second jaw 36 and the retracted position of the arm 70.

The first jaw 34 and the second jaw 36 are moved towards each other to clamp tissue therebetween. In this closed position, the arm 70 may not be actuated or can move freely so that the arm 70 does not apply pressure to the tissue and is slightly offset to the second jaw 36 defining the retracted position. In this configuration, the tissue may be sealed using microwave energy.

Subsequently, the arm 70 is actuated to be pressed towards the first electrode 54 forming the press position. In the press position, the first electrode 54 emits radiofrequency energy to cut the tissue. This means the pressure on the tissue between the first electrode 54 and the second isolating portion 74 can be selectively increased during radiofrequency cutting compared to the pressure applied during microwave sealing. At the same time as the radiofrequency cutting or after completion of the radiofrequency cutting, the first jaw 34 and the second jaw 36 may be moved away from the closed position to un-clamp the tissue allowing the cut tissue to be released. The inclination of the first face 80 and the second face 84 improves the release of the cut tissue.

The embodiment of the electrosurgical instrument 12 shown in Fig. 3 includes the same features, characteristics, and/or optional embodiment as the embodiment of the electrosurgical instrument 12 shown in Figs. 2a and 2b except for the following differences.

In this embodiment, the electrosurgical instrument 12 does not include an arm 70. Rather, the second isolating portion 74 is arranged on the second jaw 36. The third electrode 66 provides a half-shell within which the second isolating portion 74 is arranged. Exposed sections of the third electrode 66 and of the second isolating portion 74 define the second surface 52.

The second surface 52 includes a third face 90 and a fourth face 92. A line at which the third face 90 and the fourth face 92 meet may contact the first electrode 54 in the closed position. The first face 80 may be parallel to the third face 90 in the closed position. Similarly, the second face 82 may be parallel to the fourth face 92 in the closed position. So, in the closed position, a gap between the first face 80 and the third face 90 as well as the second face 82 and the fourth face 92 is constant resulting in a constant pressure over the first surface 50 and the second surface 52 except for the area of the first electrode 54. The first face 80 and the second face 82 may define an angle of 120°.

The embodiment of the electrosurgical instrument 12 shown in Fig. 4 includes the same features, characteristics, and/or optional embodiment as the embodiment of the electrosurgical instrument 12 shown in Fig. 3 except for the following differences.

An angle defined by the third face 90 and the fourth face 92 (e.g. 140° or 150°) is greater than the angle defined by the first face 80 and the second face 82 (e.g. 120°). So, in the closed position, a gap between the first face 80 and the third face 90 as well as the second face 82 and the fourth face 92 increases when moving away from the first electrode 54 resulting in a pressure decrease when moving away from the first electrode 54. This may help to increase the pressure on the tissue over the first electrode 54 for radiofrequency cutting, for example in the absence of the arm 70.

The embodiment of the electrosurgical instrument 12 shown in Fig. 5 includes the same features, characteristics, and/or optional embodiment as the embodiment of the electrosurgical instrument 12 shown in Figs. 3 and 4 except for the following differences.

An angle defined by the third face 90 and the fourth face 92 is 180° and, therefore, greater than the angle defined by the first face 80 and the second face 82 (e.g. 120°). In other words, the second surface 52 is flat. So, in the closed position, a gap between the first face 80 and the third face 90 as well as the second face 82 and the fourth face 92 increases when moving away from the first electrode 54 resulting in a pressure decrease when moving away from the first electrode 54. This may help to increase the pressure on the tissue over the first electrode 54 for radiofrequency cutting, for example in the absence of the arm.

The embodiment of the electrosurgical instrument 12 shown in Fig. 6 includes the same features, characteristics, and/or optional embodiment as the embodiment of the electrosurgical instrument 12 shown in Figs. 3 to 5 except for the following differences.

The second electrode 56 is solid having a recess in which the first isolating portion 58 is embedded. The first electrode 54 is embedded in the first isolating portion 58 while protruding therefrom. The second electrode 56 and the first isolating portion 58 provide the first surface 50 having a first curvature which is constant along the longitudinal direction of the first jaw 34 and/or perpendicular thereto.

The third electrode 66 is solid and provides a second surface 52 that has a second curvature which is constant along the longitudinal direction of the second jaw 36 and/or perpendicular thereto. The second curvature is greater than the first curvature. So, in the closed position, a gap between the first surface 50 and the second surface 52 increases when moving away from the first electrode 54 resulting in a pressure decrease when moving away from the first electrode 54. This may help to increase the pressure on the tissue over the first electrode 54 for radiofrequency cutting, for example in the absence of the arm.

Further, the second isolating portion 74 is arranged only in a region corresponding to the first electrode 54. The second isolating portion 74 protrudes from the third electrode 66 which further increases the pressure on the tissue between the first electrode 54 and the second isolating portion 74 in the closed position. This may be helpful for radiofrequency cutting as outlined above.

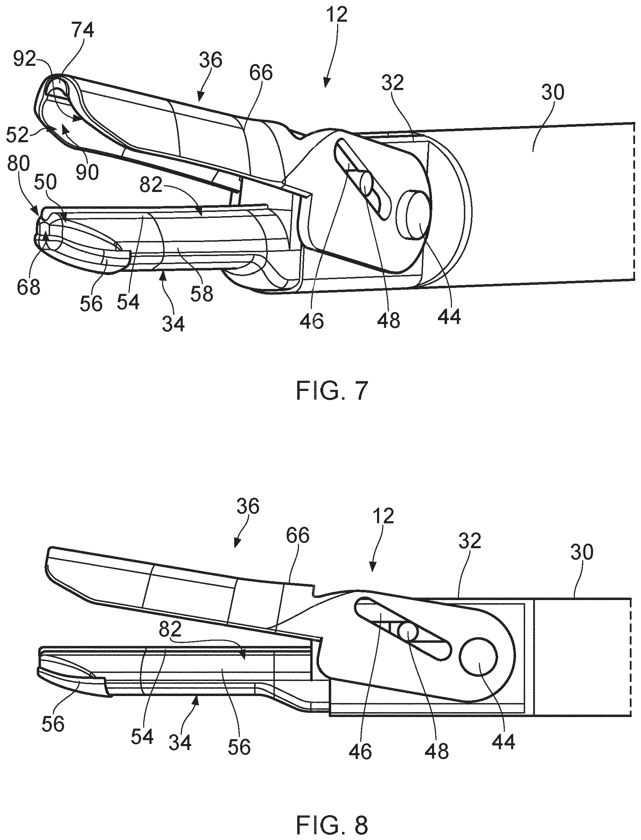

The embodiment of the electrosurgical instrument 12 shown in Figs. 7 to 9 includes the same features, characteristics, and/or optional embodiment as the embodiment of the electrosurgical instrument 12 shown in Fig. 6 except for the following differences.

The third electrode 66 includes a third face 90 and a fourth face 92 which correspond to sections of the third electrode 66 that are exposed on the second surface 52. The second surface 52 has a second curvature. The second isolating portion 74 is arranged between the third face 90 and the fourth face 92. The third electrode 66 includes a recess in which the second isolating portion 74 is embedded so that the second isolating portion 74 is flush with the third face 90 and the fourth face 92.

The first face 80 and the second face 82 have a first curvature and are formed by the first isolating portion 58. The first curvature is approximately equal to the second curvature. So, in the closed position, the first face 80 extends parallel and contacts the third face 90. Similarly, in the closed position, the second face 82 extends parallel and contacts the fourth surface 92. So, the microwave sealing is provided between the first face 80 and the third face 90 as well as between the second face 82 and the fourth face 92. Stated differently, the microwave sealing occurs between the first isolating portion 58 and the third electrode 66.

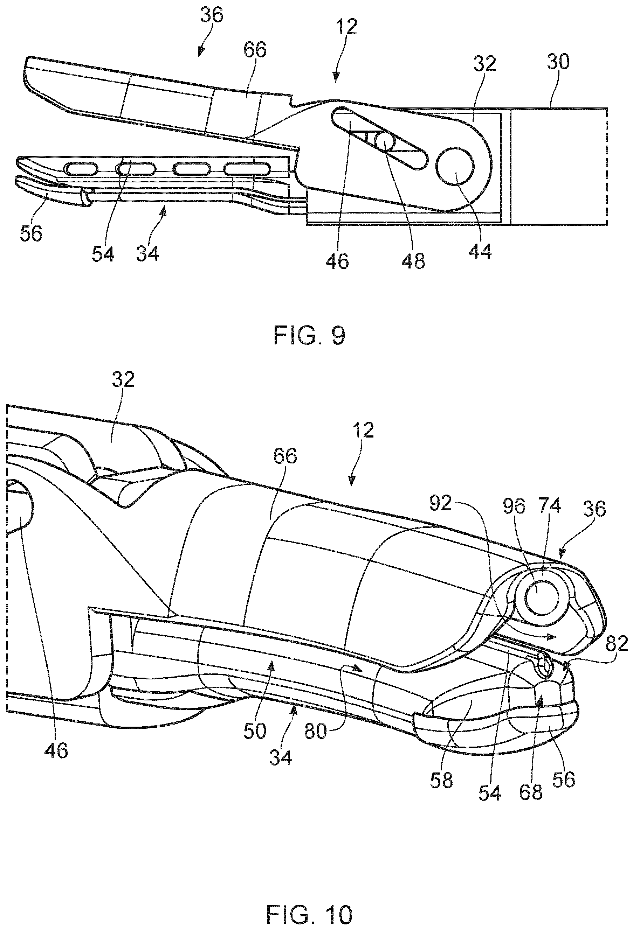

The second electrode 56 is partially embedded in the first isolating portion 58 (see Figs. 8 and 9). Only a distal end portion of first isolating portion 58 is exposed, e.g. on the distal end face 68. The first electrode 54 is also exposed on the distal end face 68. The first electrode 54 includes a supporting structure embedded in the first isolating portion 58 and a protruding ridge.

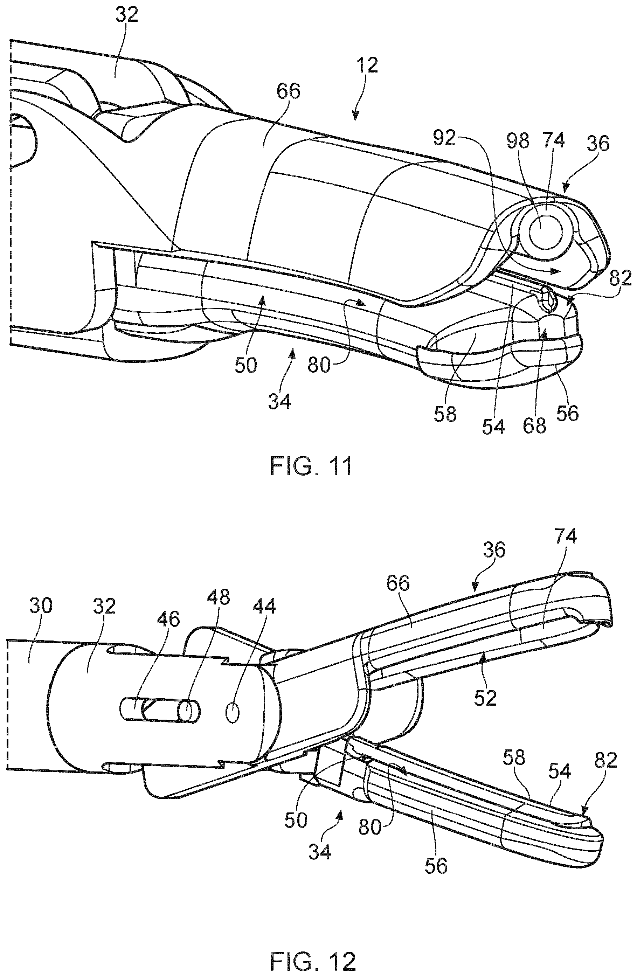

The embodiment of the electrosurgical instrument 12 shown in Figs. 10 and 11 includes the same features, characteristics, and/or optional embodiment as the embodiment of the electrosurgical instrument 12 shown in Figs. 7 to 9 except for the following differences.

The second isolating portion 74 is made from a soft or flexible material, such as silicon or silicon-based material. The second isolating portion 74 includes a lumen 96 which is a cavity that extends along the longitudinal direction of the second jaw 36. In the closed position, the first electrode 54 compresses the second isolating portion 74 so that the lumen collapses. This reduces the pressure on the tissue during the closed position which may be helpful for reducing the pressure during microwave sealing. A blocking element 98 may be inserted into the lumen 96. The blocking element 98 can include a wire made of stainless steel. The blocking element 98 expands the collapsed lumen 96 in the closed position for increasing the pressure between the first electrode 54 and the second isolating portion 74. This increases the efficiency of the radiofrequency cutting.

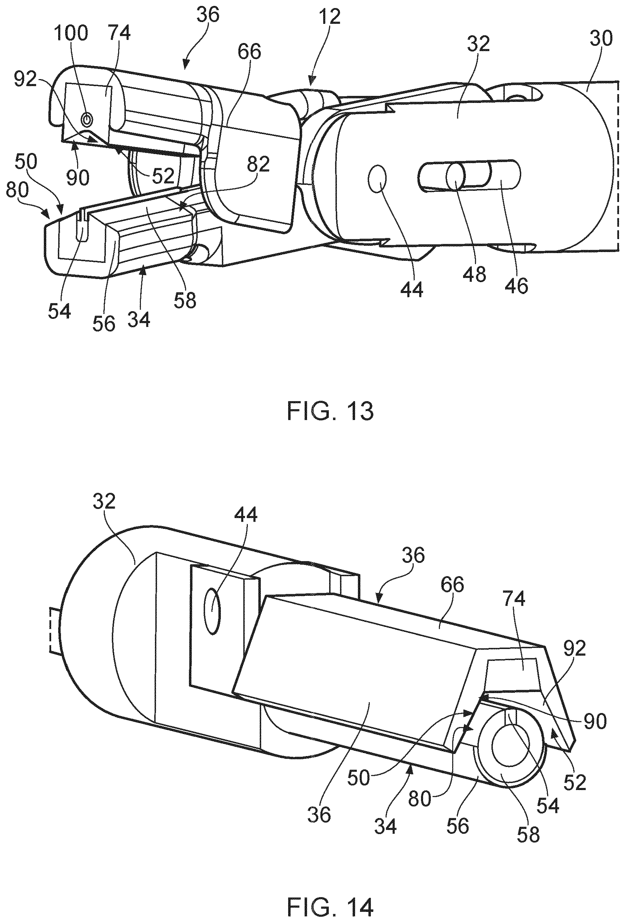

The embodiment of the electrosurgical instrument 12 shown in Figs. 12 and 13 includes the same features, characteristics, and/or optional embodiment as the embodiment of the electrosurgical instrument 12 shown in Figs. 2a and 2b except for the following differences.

The first isolating portion 56 and the second isolating portion are made from silicon. The second isolating portion 74 protrudes form the section of the third electrode 66 that is exposed on the second surface 52. The second isolating portion 74 protrudes more at a distal end of the second isolating portion 74 compared to a proximal end of the second isolating portion 74 (see Fig. 12). This helps to provide an even pressure along the longitudinal direction of the second jaw 36 in the closed position.

Further, the second isolating portion 74 includes the third face 90 and the fourth face 94 that are inclined to each other and which extend parallel to the first face 80 and the second face 82, respectively, in the closed position. The second isolating portion 74 first comes in contact and may only come in contact when moving the first jaw 34 and the second jaw 36 from the open position to the closed position.

The second jaw 36 includes a fourth electrode 100 which is embedded in the second isolating portion 74 (see Fig. 13). As such, the fourth electrode 100 is non-sticking since it is completely immersed in the non-stick silicon material of the second isolating portion 74. The fourth electrode 100 is arranged close to the second surface 52 and is configured to emit microwave energy. The second isolating portion 74 can be connected to the first electrode 54 so that microwave energy can be applied to the tissue from two opposing sides.

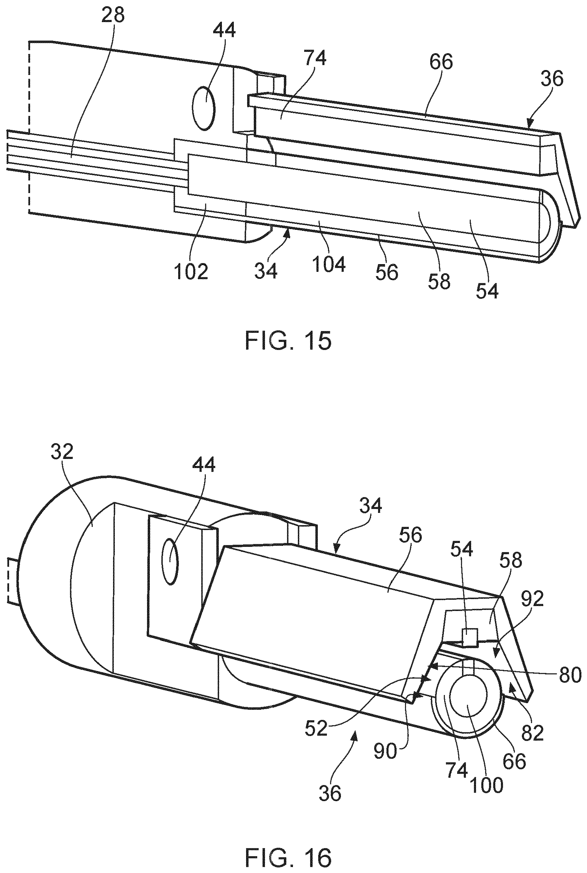

The embodiment of the electrosurgical instrument 12 shown in Figs. 14 and 15 includes the same features, characteristics, and/or optional embodiment as the embodiment of the electrosurgical instrument 12 shown in Figs. 7 to 11 except for the following differences.

The first jaw 34 is shown as having a circular cross-section. This embodiment is however not limited to this shape. Rather, the shape as shown in Fig. 7 to 11 can be applied to this embodiment.

The third electrode 66 includes the third face 90 and the fourth face 92 which correspond to sections of the third electrode 66 that are exposed on the second surface 52. The third face 90 and the fourth face 92 are each planar and define an angle of less than 180°, e.g. 120°. The second isolating portion 74 is arranged between the third face 90 and the fourth face 92.

The first face 80 and the second face 82 have a first curvature and are formed by the first isolating portion 58. In the closed position, the first face 80 contacts the third face 90. Similarly, in the closed position, the second face 82 contacts the fourth surface 92. So, the microwave sealing is provided between the first face 80 and the third face 90 as well as between the second face 82 and the fourth face 92. Stated differently, the microwave sealing occurs between the first isolating portion 58 and the third electrode 66.

The first electrode 54 is embedded in the first isolating portion 58 except for the part of the first electrode 54 that is exposed on the first surface 50. The first electrode 54 is flush with the first isolating portion 58 on the first surface 50, i.e. it does not protrude from the first isolating portion 58.

The first isolating portion 58 includes a first material portion 102 (e.g. made from Zirconia or Alumina) and a second material portion 104 (e.g. made from silicon). The first material portion 102 is arranged between the transmission line and the second material portion 104. The material portion 102 is in contact with an isolator of a coaxial cable of the transmission line. The second material portion 104 is the only part of the first isolating portion 58 that is exposed on the first surface 50.

The first material portion 102 acts as a transformer to match (e.g. impedance match) a microwave antenna provided by the first electrode 54 and the second electrode 56. Essentially, changing the dielectric material changes the dielectric constant which in turn changes the impedance of that section. Then the transformer may need to have the correct length (typically ¼ wavelength or odd multiple) to match at the specified frequency. There is also possibility to use a half wavelength.

The first jaw 34 is fixed with respect to the shaft 30. Only the second jaw 36 is movable relative to the shaft 30.

The embodiment of the electrosurgical instrument 12 shown in Fig. 16 includes the same features, characteristics, and/or optional embodiment as the embodiment of the electrosurgical instrument 12 shown in Figs. 12 and 13 except for the following differences.

It is to be noted that, in Fig. 16, the first jaw 34 is the upper jaw and the second jaw is the lower jaw. This orientation is the opposite of all other figures.

The second electrode 56 includes the first face 80 and the second face 82 which correspond to sections of the second electrode 56 that are exposed on the first surface 50. The first face 80 and the second face 82 are each planar and define an angle of less than 180°, e.g. 120°. The first isolating portion 58 is arranged between the first face 80 and the second face 82. The first electrode 54 protrudes from the first isolating portion 58.

The third face 90 and the fourth face 92 have a second curvature and are formed by the second isolating portion 74. In the closed position, the first face 80 contacts the third surface 90. Similarly, in the closed position, the second face 82 contacts the fourth surface 92. So, the microwave sealing is provided between the first face 80 and the third face 90 as well as between the second face 82 and the fourth face 92. Stated differently, the microwave sealing occurs between the second isolating portion 74 and the second electrode 56.

The fourth electrode 100 is completely embedded in the second isolating portion 74. The fourth electrode 100 is again provided for emitting microwave energy.

The second jaw 36 is fixed with respect to the shaft 30. Only the first jaw 34 is movable relative to the shaft 36.

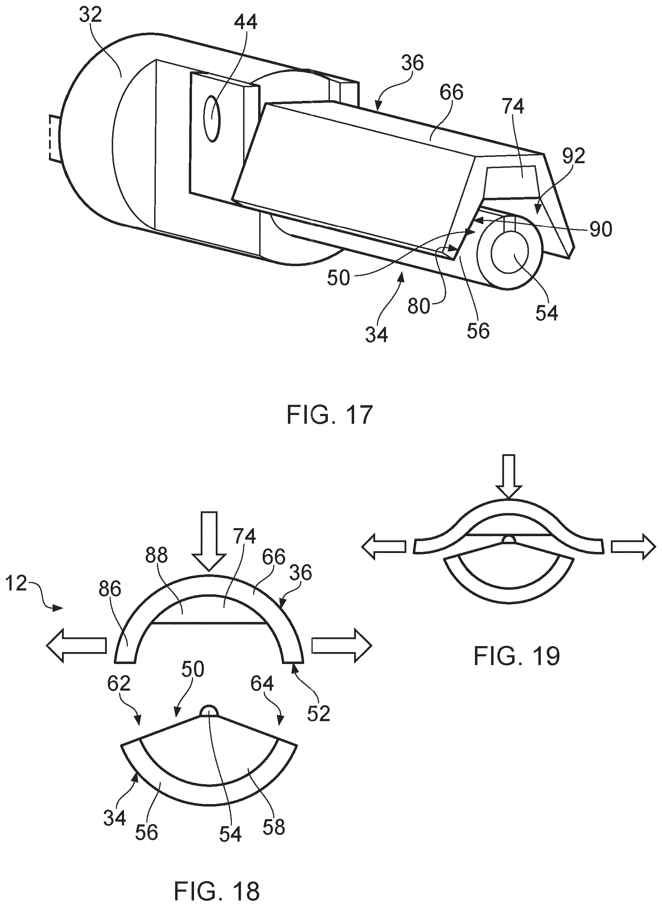

The embodiment of the electrosurgical instrument 12 shown in Fig. 17 includes the same features, characteristics, and/or optional embodiment as the embodiment of the electrosurgical instrument 12 shown in Figs. 14 and 15 except for the following differences.

The first jaw 34 does not include a second electrode 56. Thus, the first jaw 34 of the electrosurgical instrument 12 shown in Fig. 17 is not configured to emit microwave energy or radiofrequency energy on its own. Rather, the emission of microwave energy or radiofrequency energy is only possible in connection with the third electrode 66 on the second jaw 36.

The embodiment of the electrosurgical instrument 12 shown in Figs. 18 and 19 includes the same features, characteristics, and/or optional embodiment as the embodiments of the electrosurgical instrument 12 shown in Figs. 7 to 9 and except for the following differences.

The second jaw 36 includes a protruding portion 86 and a recessed portion 88. The protruding portion 86 can be made from a flexible material, such as a plastic material. The protruding portion 86 can be covered by an electrically conductive layer for providing the fourth electrode 66. Distal end faces of the protruding portion 86 define the second surface 52.

The recessed portion 88 may be provided by the second isolating portion 74. In the closed position, the recessed portion 88 does not contact the first electrode 54, i.e. there is a gap between first electrode 54 and the recessed portion 88. Thus, in the closed position, little or no pressure is applied to the tissue between the first electrode 54 and the recessed portion 88 compared to the pressure applied to the tissue between the first surface 50 and the second surface 52 where the microwave sealing takes place.

As illustrated in Fig. 19, due to the flexibility of the protruding portion 86, the second jaw 36 can be further moved towards the first jaw 34 so that pressure can be applied on the tissue between the first electrode 54 and the recessed portion 88 for effecting radiofrequency cutting. The protruding portion 86 may bend outwards when the second jaw 36 is moved towards the first jaw 34 beyond the closed position as indicated by the arrows in Figs. 18 and 19.

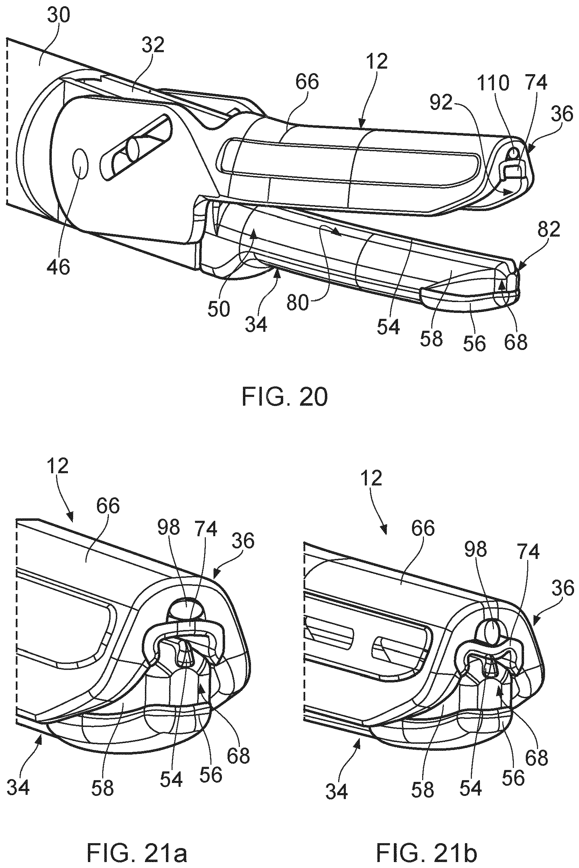

The embodiment of the electrosurgical instrument 12 shown in Figs. 20, 21a, and 21b includes the same features, characteristics, and/or optional embodiment as the embodiment of the electrosurgical instrument 12 shown in Figs. 10 and 11 except for the following differences.

The second jaw 36 includes a channel 110 that is defined between and by the second isolating portion 74 and the third electrode 66. The second isolating portion 74 may be elastic and/or is formed like a membrane. The second isolating portion 74 may cover a groove in the third electrode 66.

The blocking element 98 has a maximal diameter and a minimal diameter in a cross-sectional view of the blocking element 98 and/or the second jaw 36. In a first orientation, the maximal diameter is horizontal and the minimal diameter is vertical in Figs. 20 and 21a. The channel 110 includes a first (maximal) diameter and a second (minimal) diameter. The first diameter is horizontal and the second diameter is vertical in the orientation shown in Figs. 20 and 21a. The first diameter is greater than the maximal diameter of the blocking element 98 and the second diameter is larger than the minimal diameter of the blocking element 98 so that the blocking element 98 fits within the channel 110 without deforming, bending, or bulging the second isolation portion 74. In the closed position and the first orientation of the blocking element 98, the second isolating portion 74 does not contact the first electrode 54.

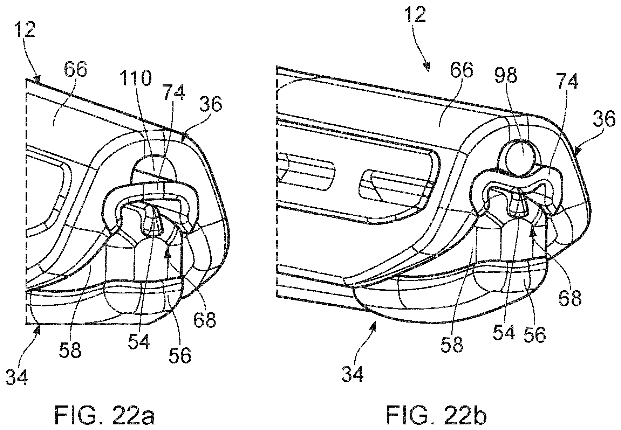

By rotating the blocking element 98 by 90°, the maximal diameter of the blocking element 98 is vertical (see Fig. 21b) and thus parallel to the second diameter of the channel 110. The minimal diameter of the blocking element 98 is horizontal (see Fig. 21b) and thus parallel to the first diameter of the channel 110. The maximal diameter of the blocking element 98 is greater than the second diameter of the channel 110 so that the second isolation portion 74 is deformed, bent, or bulged towards the first electrode 54. Thus, by rotating the blocking element 98, pressure on the tissue between the first electrode 54 and the second isolation portion 74 can be increased. The embodiment of the electrosurgical instrument 12 shown in Figs. 22a and 22b includes the same features, characteristics, and/or optional embodiment as the embodiment of the electrosurgical instrument 12 shown in Figs. 20, 21a, and 21b except for the following differences.

The blocking element 98 has a larger cross-sectional area compared to a cross-sectional area of the blocking element 98. In the absence of the blocking element 98, no pressure is applied on the second isolation portion 74. When the blocking element 98 is inserted into the channel 110, the second isolation portion 74 is deformed, bent, or bulged towards the first electrode 54 in the closed position. This increases the pressure on the tissue between the first electrode 54 and the second isolation portion 74.

## Claims

1. An electrosurgical instrument for sealing and/or cutting tissue, comprising
an instrument shaft (30) comprising a transmission line for conveying microwave and/or radiofrequency electromagnetic energy;
a first jaw (34) attached to the instrument shaft (30) and including a first surface (50),
a second jaw (36) attached to the instrument shaft (30) and including a second surface (52),
a first electrode (54) for emitting microwave and radiofrequency energy,
a second electrode (56) for emitting microwave and/or radiofrequency energy,
a third electrode (66) for emitting microwave and/or radiofrequency energy,
a first isolating portion (58) electrically isolating the first electrode (54) from the second electrode (56), and
a second isolating portion (74) which is arranged on the second jaw (36),
wherein the first jaw (34) and the second jaw (36) can be moved between an open position, in which the tissue can be inserted between the first surface (50) and the second surface (52) (52), and a closed position, in which the first and second surfaces (50, 52) are brought together to clamp the tissue therebetween,
wherein the first electrode (54) and the second electrode (56) are arranged on the first jaw (34),
wherein the first electrode (54) protrudes from the first isolating portion (58),
wherein the third electrode (66) is arranged on the second jaw (36), and
wherein the second electrode (56) and the third electrode (66) are arranged on opposing sides of the first electrode (54) in the closed position and are electrically connected to each other,
**characterized in that** the second isolating portion (74) protrudes more from the third electrode (66) at a distal end of the second surface (52) compared to a proximal end of the second surface (52).

2. The electrosurgical instrument of claim 1, wherein a first face (80) of the first isolating portion (58) and/or the second electrode (56) and a second face (84) of the first isolating portion (58) and/or the second electrode (56) are arranged on opposing sides of the first electrode (54), wherein the first face (80) and the second face (84) define an angle of less than 180° with respect to each other,
wherein optionally the second surface (52) includes a third face (90) and a fourth face (92),
wherein
the first face (80) is parallel to the third face (90) and the second face (84) is parallel to the fourth face (92), or
the angle defined by the first face (80) and the second face (84) is smaller than an angle defined by the third face (90) and the fourth face (92), or
the third face (90) and the fourth face (92) define an angle of 180° with each other.

3. The electrosurgical instrument of claim 1, wherein the first surface (50) includes a first curvature in a cross-sectional view of the first jaw (34) and the second surface (52) includes a second curvature in a cross-sectional view of the second jaw (36), wherein the second curvature is smaller than the first curvature.

4. The electrosurgical instrument of claims 2 or 3, wherein
the third electrode (66) forms the third face (90) and the fourth face (92), the second isolating portion (74) being arranged between the third face (90) and the fourth face (92), or
the third electrode (66) provides the second curvature, the second isolating portion (74) being flush with or protruding from the second curvature, or
the second isolating portion (74) forms the third face (90) and the fourth face (92), the second isolating portion (74) protruding from the third electrode (66), optionally only the second isolating portion (74) is in contact with the first surface (50) in the closed position, or
wherein the second electrode (56) forms a first face (80) and a second face (84), the first isolating portion (58) being arranged between the first face (80) and the second face (84),
wherein optionally
the electrosurgical instrument further comprises a fourth electrode (100), wherein the fourth electrode (100) is embedded in the second isolating portion (74), or
the second isolating portion (74) includes a lumen (96) and the lumen (96) is configured to be deformed in the closed position, wherein optionally the electrosurgical instrument (12) comprises a blocking element (98) which is insertable into the lumen (96) for filling the lumen (96).

5. The electrosurgical instrument of claim 4, further comprising a blocking element (98), which is insertable into the channel,
wherein the second isolating portion (74) and the third electrode (66) are arranged to define a channel therebetween, the second isolating portion (74) being elastic,
wherein an area of the channel in a cross-sectional view of the second jaw (36) is smaller than an area of the blocking element (98) in a cross-sectional view of the blocking element (98) so that the second isolating portion (74) is deformed towards the first electrode (54) in the closed position, or
wherein the blocking element (98) includes a minimal diameter and a maximal diameter in a cross-sectional view of the blocking element (98), the channel including a first diameter, which is larger the maximal diameter of the blocking element (98), and a second diameter, which is greater than the minimal diameter and smaller than the maximal diameter, so that a rotation of the blocking element (98) is configured to deform the second isolating portion (74) towards the first electrode (54) in the closed position.

6. The electrosurgical instrument of any one of the claims 1 to 3, wherein the second jaw (36) includes a protruding portion (86) and a recessed portion (88), wherein, in the closed position, the protruding portion (86) is in contact with the first surface (50) and the recessed portion (88) is spaced from the first electrode (54), and wherein the protruding portion (86) is flexible so that the second jaw (36) can be further moved towards the first surface (50) relative to the closed position so that the recessed portion (88) can be moved closer towards the first electrode (54).

7. The electrosurgical instrument of any preceding claim, wherein the second electrode (56) covers the first electrode (54) on the side of the first electrode (54) facing away from the first surface (50) and/or wherein the first isolating portion (58) is arranged between the first electrode (54) and the second electrode (56).

8. The electrosurgical instrument of any preceding claim, wherein a portion of the second electrode (56) is plate-shaped and includes end faces, wherein the end faces form sections of the second electrode (56) that are exposed on the first surface (50), wherein, in a cross-sectional view of the first jaw (34), the portions of the second electrode (56) are U-shaped or V-shaped,
wherein optionally the sections of the second electrode (56) that are exposed on the first surface (50) at least partially extend parallel to each other and/or with the first electrode (54) arranged therebetween.

9. The electrosurgical instrument of any one of the claims 1 to 6, wherein a part of the second electrode (56) is embedded in the first isolating portion (58), wherein optionally a distal end portion of the second electrode (56) is exposed on the first isolating portion (58).

10. The electrosurgical instrument of any preceding claim, wherein the first jaw (34) includes a distal end face (68), wherein the first electrode (54), the second electrode (56), and/or the first isolating portion (58) are exposed on the distal end face (68).

11. The electrosurgical instrument of any preceding claim, further comprising a joint (32) having a pivot axis and an arm (70) movable relative to the second jaw (36),
wherein the arm (70) is configured to be moved between a press position, in which the arm (70) and the first surface (50) are brought together to press the tissue therebetween, and a retracted position, in which the arm (70) is spaced away from the first surface (50),
wherein optionally the second jaw (36) is rotatable around a pivot axis of the joint (32), wherein the arm (70) is also rotatable around the pivot axis, and
wherein further optionally
the second jaw (36) and the arm (70) are configured to be rotated independently from each other, or
the second jaw (36) includes a jaw slot (84) and the arm (70) includes an arm cam (86), wherein the arm cam (86) is inserted in the jaw slot (84) for providing movement of the arm (70) relative to the second jaw (36) between the retracted position and the press position.

12. The electrosurgical instrument of claim 11, wherein the second jaw (36) includes an opening through which the arm (70) can be moved, and/or
wherein the arm (70) includes a second isolating portion (74) which is configured to be in contact with the first electrode (54) in the press position of the arm (70), and/or
wherein the arm (70) and the second jaw (36) respectively include conductive materials which form the third electrode (66), and/or
the second jaw (36) includes a third isolating portion which covers the opening and is flexible across the opening so that the arm (70) is configured to deform the third isolating portion in the closed position or the arm (70) deforms the third isolating portion in a press position of the arm (70), in which the arm (70) and the first surface (50) are brought together to cut tissue therebetween, wherein optionally the third isolating portion is made from silicon.

13. The electrosurgical instrument of any preceding claim, wherein the first isolating portion (58) includes a first material portion (102) and a second material portion (104), wherein the first material portion (102) is arranged between the transmission line and the second material portion (104), and wherein a material of the first material portion (102) is different to a material of the second material portion (104).

14. An electrosurgical apparatus for sealing and cutting tissue, comprising
a generator unit (14) for generating radiofrequency and/or microwave electromagnetic energy, and
the electrosurgical instrument (12) according to any preceding claim,
wherein the transmission line is configured to convey the radiofrequency and/or microwave electromagnetic energy from the generator unit (14) to the first electrode (54), the second electrode (56) and/or the third electrode (66).

15. The electrosurgical apparatus of claim 14, wherein the generator unit (14) is configured to simultaneously or alternatingly generate microwave electromagnetic energy of a first frequency and radiofrequency electromagnetic energy of a second frequency.

## Patentansprüche

1. Elektrochirurgisches Instrument zum Versiegeln und/oder Schneiden von Gewebe, das Folgendes umfasst:
einen Instrumentenschaft (30), der eine Übertragungsleitung zum Transport von elektromagnetischer Mikrowellen- und/oder Hochfrequenzenergie umfasst;
eine erste Backe (34), die am Instrumentenschaft (30) befestigt ist und eine erste Oberfläche (50) umfasst,
eine zweite Backe (36), die am Instrumentenschaft (30) befestigt ist und eine zweite Oberfläche (52) umfasst,
eine erste Elektrode (54) zur Emission von Mikrowellen- und Hochfrequenzenergie,
eine zweite Elektrode (56) zur Emission von Mikrowellen- und/oder Hochfrequenzenergie,
eine dritte Elektrode (66) zur Emission von Mikrowellen- und/oder Hochfrequenzenergie,
einen ersten Isolationsabschnitt (58), der die erste Elektrode (54) elektrisch von der zweiten Elektrode (56) isoliert, und
einen zweiten Isolationsabschnitt (74), der auf der zweiten Backe (36) angeordnet ist,
wobei die erste Backe (34) und die zweite Backe (36) zwischen einer offenen Stellung, in der das Gewebe zwischen die erste Oberfläche (50) und die zweite Oberfläche (52) eingeführt werden kann, und einer geschlossenen Stellung, in der die erste und zweite Oberfläche (50, 52) zusammengebracht sind, um das Gewebe dazwischen einzuklemmen, bewegt werden kann,
wobei die erste Elektrode (54) und die zweite Elektrode (56) auf der ersten Backe (34) angeordnet sind,
wobei die erste Elektrode (54) vom ersten Isolationsabschnitt (58) vorsteht,
wobei die dritte Elektrode (66) auf der zweiten Backe (36) angeordnet ist und
wobei die zweite Elektrode (56) und die dritte Elektrode (66) in der offenen Stellung auf gegenüberliegenden Seiten der ersten Elektrode (54) angeordnet sind und elektrisch miteinander verbunden sind,
**dadurch gekennzeichnet, dass** der zweite Isolationsabschnitt (74) am distalen Ende der zweiten Oberfläche (52) mehr von der dritten Elektrode (66) vorsteht als am proximalen Ende der zweiten Oberfläche (52).

2. Elektrochirurgisches Instrument nach Anspruch 1, wobei eine erste Fläche (80) des ersten Isolationsabschnitts (58) und/oder der zweiten Elektrode (56) und eine zweite Fläche (84) des ersten Isolationsabschnitts (58) und/oder der zweiten Elektrode (56) auf gegenüberliegenden Seiten der ersten Elektrode (54) angeordnet sind, wobei die erste Fläche (80) und die zweite Fläche (84) einen Winkel von weniger als 180° zueinander definieren,
wobei die zweite Oberfläche (52) gegebenenfalls eine dritte Fläche (90) und eine vierte Fläche (92) umfasst,
wobei
die erste Fläche (80) parallel zur dritten Fläche (90) ist und die zweite Fläche (84) parallel zur vierten Fläche (92) ist oder
der von der ersten Fläche (80) und der zweiten Fläche (84) definierte Winkel kleiner ist als ein Winkel, der durch die dritte Fläche (90) und die vierte Fläche (92) definiert ist, oder
die dritte Fläche (90) und die vierte Fläche (92) einen Winkel von 180° zueinander definieren.

3. Elektrochirurgisches Instrument nach Anspruch 1, wobei die erste Oberfläche (50) in einer Querschnittsansicht der ersten Backe (34) eine erste Krümmung umfasst und die zweite Oberfläche (52) in einer Querschnittsansicht der zweiten Backe (36) eine zweite Krümmung umfasst, wobei die zweite Krümmung kleiner ist als die erste Krümmung.

4. Elektrochirurgisches Instrument nach Anspruch 2 oder 3, wobei
die dritte Elektrode (66) die dritte Fläche (90) und die vierte Fläche (92) ausbildet, wobei der zweite Isolationsabschnitt (74) zwischen der dritten Fläche (90) und der vierten Fläche (92) angeordnet ist, oder
die dritte Elektrode (66) die zweite Krümmung bereitstellt, wobei der zweite Isolationsabschnitt (74) mit der zweiten Krümmung bündig ist oder von der zweiten Krümmung vorsteht, oder
der zweite Isolationsabschnitt (74) die dritte Fläche (90) und die vierte Fläche (92) ausbildet, wobei der zweite Isolationsabschnitt (74) von der dritten Elektrode (66) vorsteht, wobei in der geschlossenen Stellung gegebenenfalls nur der zweite Isolationsabschnitt (74) mit der ersten Oberfläche (50) in Kontakt ist, oder
wobei die zweite Elektrode (56) eine erste Fläche (80) und eine zweite Fläche (84) ausbildet, wobei der erste Isolationsabschnitt (58) zwischen der ersten Fläche (80) und der zweiten Fläche (84) angeordnet ist,
wobei gegebenenfalls
das elektrochirurgische Instrument weiters eine vierte Elektrode (100) umfasst, wobei die vierte Elektrode (100) im zweiten Isolationsabschnitt (74) eingebettet ist, oder
der zweite Isolationsabschnitt (74) ein Lumen (96) umfasst und das Lumen (96) ausgeausbildet ist, in der geschlossenen Stellung verformt zu sein, wobei das elektrochirurgische Instrument (12) gegebenenfalls ein Blockierelement (98) umfasst, das in das Lumen (96) einführbar ist, um das Lumen (96) zu füllen.

5. Elektrochirurgisches Instrument nach Anspruch 4, das weiters ein Blockierelement (98) umfasst, das in den Kanal einführbar ist,
wobei der zweite Isolationsabschnitt (74) und die dritte Elektrode (66) angeordnet sind, um einen Kanal dazwischen zu definieren, wobei der zweite Isolationsabschnitt (74) elastisch ist,
wobei ein Bereich des Kanals in einer Querschnittsansicht der zweiten Backe (36) kleiner ist als ein Bereich des Blockierelements (98) in einer Querschnittsansicht des Blockierelements (98), sodass der zweite Isolationsabschnitt (74) in der geschlossenen Stellung zur ersten Elektrode (54) hin verformt ist, oder
wobei das Blockierelement (98) in einer Querschnittsansicht des Blockierelements (98) einen Mindestdurchmesser und einen Maximaldurchmesser umfasst, wobei der Kanal einen ersten Durchmesser, der größer ist als der Maximaldurchmesser des Blockierelements (98), und einen zweiten Durchmesser, der größer als der Mindestdurchmesser und kleiner als der Maximaldurchmesser ist, umfasst, sodass eine Rotation des Blockierelements (98) ausgelegt ist, den zweiten Isolationsabschnitt (74) in der geschlossenen Stellung zur ersten Elektrode (54) hin zu verformen.

6. Elektrochirurgisches Instrument nach einem der Ansprüche 1 bis 3, wobei die zweite Backe (36) einen vorspringenden Abschnitt (86) und einen zurückgesetzten Abschnitt (88) umfasst, wobei in der geschlossenen Stellung der vorspringende Abschnitt (86) mit der ersten Oberfläche (50) in Kontakt ist und der zurückgesetzte Abschnitt (88) von der ersten Elektrode (54) beabstandet ist und wobei der vorspringende Abschnitt (86) flexibel ist, sodass die zweite Backe (36) relativ zur geschlossenen Stellung weiter zur ersten Oberfläche (50) hin bewegt werden kann, sodass der zurückgesetzte Abschnitt (88) näher zur ersten Elektrode (54) hin bewegt werden kann.

7. Elektrochirurgisches Instrument nach einem der vorangegangenen Ansprüche, wobei die zweite Elektrode (56) die erste Elektrode (54) auf der Seite der ersten Elektrode (54), die von der ersten Oberfläche (50) weg weist, abdeckt und/oder wobei der erste Isolationsabschnitt (58) zwischen der ersten Elektrode (54) und der zweiten Elektrode (56) angeordnet ist.

8. Elektrochirurgisches Instrument nach einem der vorangegangenen Ansprüche, wobei ein Abschnitt der zweiten Elektrode (56) plattenförmig ist und Endflächen umfasst, wobei die Endflächen Teilabschnitte der zweiten Elektrode (56) ausbilden, die auf der ersten Oberfläche (50) freigelegt sind, wobei die Abschnitte der zweiten Elektrode (56) in einer Querschnittsansicht der ersten Backe (34) U-förmig oder V-förmig sind,
wobei die Teilabschnitte der zweiten Elektrode (56), die auf der ersten Oberfläche (50) freigelegt sind, sich gegebenenfalls zumindest teilweise parallel zueinander und/oder zur ersten Elektrode (54), die dazwischen angeordnet ist, erstrecken.

9. Elektrochirurgisches Instrument nach einem der Ansprüche 1 bis 6, wobei ein Abschnitt der zweiten Elektrode (56) im ersten Isolationsabschnitt (58) eingebettet ist, wobei gegebenenfalls ein distaler Endabschnitt der zweiten Elektrode (56) auf dem ersten Isolationsabschnitt (58) freigelegt ist.

10. Elektrochirurgisches Instrument nach einem der vorangegangenen Ansprüche, wobei die erste Backe (34) eine distale Endfläche (68) umfasst, wobei die erste Elektrode (54), die zweite Elektrode (56) und/oder der erste Isolationsabschnitt (58) auf der distalen Endfläche (68) freigelegt sind.

11. Elektrochirurgisches Instrument nach einem der vorangegangenen Ansprüche, das weiters ein Gelenk (32) mit einer Schwenkachse und einem Arm (70) umfasst, der relativ zur zweiten Backe (36) bewegbar ist,
wobei der Arm (70) ausgelegt ist, zwischen einer Pressstellung, in welcher der Arm (70) und die erste Oberfläche (50) zusammengebracht sind, um das Gewebe dazwischen zusammenzupressen, und einer zurückgezogenen Stellung, in welcher der Arm (70) von der ersten Oberfläche (50) beabstandet ist, bewegt zu werden,
wobei die zweite Backe (36) gegebenenfalls um eine Schwenkachse des Gelenks (32) rotierbar ist, wobei der Arm (70) auch um die Schwenkachse rotierbar ist, und
wobei weiters gegebenenfalls
die zweite Backe (36) und der Arm (70) ausgelegt sind, unabhängig voneinander rotiert zu werden, oder
die zweite Backe (36) einen Backenschlitz (84) umfasst und der Arm (70) eine Armnocke (86) umfasst, wobei die Armnocke (86) in den Backenschlitz (84) eingeführt wird, um eine Bewegung des Arms (70) relativ zur zweiten Backe (36) zwischen der zurückgezogenen Stellung und der Pressstellung bereitzustellen.

12. Elektrochirurgisches Instrument nach Anspruch 11, wobei die zweite Backe (36) eine Öffnung umfasst, durch die der Arm (70) bewegt werden kann, und/oder
wobei der Arm (70) einen zweiten Isolationsabschnitt (74) umfasst, der ausgelegt ist, um in der Pressstellung des Arms (70), mit der ersten Elektrode (54) in Kontakt zu sein, und/oder
wobei der Arm (70) und die zweite Backe (36) jeweils leitende Materialien umfassen, welche die dritte Elektrode (66) ausbilden, und/oder
die zweite Backe (36) einen dritten Isolationsabschnitt umfasst, der die Öffnung abdeckt und flexibel über die Öffnung ist, sodass der Arm (70) ausgelegt ist, den dritten Isolationsabschnitt in der geschlossenen Stellung zu verformen, oder der Arm (70) den dritten Isolationsabschnitt in einer Pressstellung des Arms (70) verformt, in welcher der Arm (70) und die erste Oberfläche (50) zusammengebracht werden, um Gewebe dazwischen zu schneiden, wobei der dritte Isolationsabschnitt gegebenenfalls aus Silizium besteht.

13. Elektrochirurgisches Instrument nach einem der vorangegangenen Ansprüche, wobei der erste Isolationsabschnitt (58) einen ersten Materialabschnitt (102) und einen zweiten Materialabschnitt (104) umfasst, wobei der erste Materialabschnitt (102) zwischen der Übertragungsleitung und dem zweiten Materialabschnitt (104) angeordnet ist und wobei sich das Material des ersten Materialabschnitts (102) vom Material des zweiten Materialabschnitts (104) unterscheidet.

14. Elektrochirurgisches Gerät zum Versiegeln und Schneiden von Gewebe, das Folgendes umfasst:
eine Generatoreinheit (14) zum Erzeugen von elektromagnetischer Hochfrequenz- und/oder Mikrowellenergie und
ein elektrochirurgisches Instrument (12) nach einem der vorangegangenen Ansprüche,
wobei die Übertragungsleitung ausgelegt ist, die elektromagnetische Hochfrequenz- und/oder Mikrowellenenergie von der Generatoreinheit (14) zur ersten Elektrode (54), zweiten Elektrode (56) und/oder dritten Elektrode (66) zu transportieren.

15. Elektrochirurgisches Gerät nach Anspruch 14, wobei die Generatoreinheit (14) ausgelegt ist, gleichzeitig oder abwechselnd elektromagnetische Mikrowellenenergie mit einer ersten Frequenz und elektromagnetische Hochfrequenzenergie mit einer zweiten Frequenz zu erzeugen.

## Revendications

1. Instrument électrochirurgical pour refermer et/ou couper un tissu, comprenant
une tige d'instrument (30) comprenant une ligne de transmission pour transporter de l'énergie électromagnétique hyperfréquence et/ou radiofréquence ;
une première mâchoire (34) fixée à la tige d'instrument (30) et incluant une première surface (50),
une seconde mâchoire (36) fixée à la tige d'instrument (30) et incluant une seconde surface (52),
une première électrode (54) pour émettre de l'énergie hyperfréquence et radiofréquence,
une deuxième électrode (56) pour émettre de l'énergie hyperfréquence et/ou radiofréquence,
une troisième électrode (66) pour émettre de l'énergie hyperfréquence et/ou radiofréquence,
une première partie d'isolation (58) isolant électriquement la première électrode (54) de la deuxième électrode (56), et
une deuxième partie d'isolation (74) qui est agencée sur la seconde mâchoire (36),
dans lequel la première mâchoire (34) et la seconde mâchoire (36) peuvent être déplacées entre une position ouverte, dans laquelle le tissu peut être inséré entre la première surface (50) et la seconde surface (52), et une position fermée, dans laquelle les première et seconde surfaces (50, 52) sont rapprochées pour serrer le tissu entre celles-ci,
dans lequel la première électrode (54) et la deuxième électrode (56) sont agencées sur la première mâchoire (34),
dans lequel la première électrode (54) fait saillie à partir de la première partie d'isolation (58),
dans lequel la troisième électrode (66) est agencée sur la seconde mâchoire (36), et
dans lequel la deuxième électrode (56) et la troisième électrode (66) sont agencées sur des côtés opposés de la première électrode (54) dans la position fermée et sont connectées électriquement l'une à l'autre,
**caractérisé en ce que** la deuxième partie d'isolation (74) dépasse davantage à partir de la troisième électrode (66) au niveau d'une extrémité distale de la seconde surface (52) par rapport à une extrémité proximale de la seconde surface (52).

2. Instrument électrochirurgical selon la revendication 1, dans lequel une première face (80) de la première partie d'isolation (58) et/ou de la deuxième électrode (56) et une deuxième face (84) de la première partie d'isolation (58) et/ou de la deuxième électrode (56) sont agencées sur des côtés opposés de la première électrode (54), dans lequel la première face (80) et la deuxième face (84) définissent un angle inférieur à 180° l'une par rapport à l'autre,
dans lequel facultativement la seconde surface (52) inclut une troisième face (90) et une quatrième face (92),
dans lequel
la première face (80) est parallèle à la troisième face (90) et la deuxième face (84) est parallèle à la quatrième face (92), ou l'angle défini par la première face (80) et la deuxième face (84) est inférieur à un angle défini par la troisième face (90) et la quatrième face (92), ou
la troisième face (90) et la quatrième face (92) définissent un angle de 180° l'une avec l'autre.

3. Instrument électrochirurgical selon la revendication 1, dans lequel la première surface (50) inclut une première courbure dans une vue en coupe transversale de la première mâchoire (34) et la seconde surface (52) inclut une seconde courbure dans une vue en coupe transversale de la seconde mâchoire (36), dans lequel la seconde courbure est plus petite que la première courbure.

4. Instrument électrochirurgical selon la revendication 2 ou 3, dans lequel :
la troisième électrode (66) forme la troisième face (90) et la quatrième face (92), la deuxième partie d'isolation (74) étant agencée entre la troisième face (90) et la quatrième face (92), ou
la troisième électrode (66) fournit la seconde courbure, la deuxième partie d'isolation (74) affleurant ou faisant saillie à partir de la seconde courbure, ou
la deuxième partie d'isolation (74) forme la troisième face (90) et la quatrième face (92), la deuxième partie d'isolation (74) faisant saillie à partir de la troisième électrode (66), facultativement seule la deuxième partie d'isolation (74) est en contact avec la première surface (50) dans la position fermée, ou
dans lequel la deuxième électrode (56) forme une première face (80) et une deuxième face (84), la première partie d'isolation (58) étant agencée entre la première face (80) et la deuxième face (84),
dans lequel facultativement
l'instrument électrochirurgical comprend en outre une quatrième électrode (100), dans lequel la quatrième électrode (100) est incorporée dans la deuxième partie d'isolation (74), ou
la deuxième partie d'isolation (74) inclut une lumière (96) et la lumière (96) est configurée pour être déformée dans la position fermée, dans lequel, facultativement, l'instrument électrochirurgical (12) comprend un élément de blocage (98) qui peut être inséré dans la lumière (96) pour remplir la lumière (96).

5. Instrument électrochirurgical selon la revendication 4, comprenant en outre un élément de blocage (98), qui peut être inséré dans le canal,
dans lequel la deuxième partie d'isolation (74) et la troisième électrode (66) sont agencées pour définir un canal entre celles-ci, la deuxième partie d'isolation (74) étant élastique,
dans lequel une aire du canal dans une vue en coupe transversale de la seconde mâchoire (36) est inférieure à une aire de l'élément de blocage (98) dans une vue en coupe transversale de l'élément de blocage (98) de sorte que la deuxième partie d'isolation (74) est déformée vers la première électrode (54) dans la position fermée, ou
dans lequel l'élément de blocage (98) inclut un diamètre minimal et un diamètre maximal dans une vue en coupe transversale de l'élément de blocage (98), le canal incluant un premier diamètre, qui est plus grand que le diamètre maximal de l'élément de blocage (98), et un second diamètre, qui est plus grand que le diamètre minimal et plus petit que le diamètre maximal, de sorte qu'une rotation de l'élément de blocage (98) est configurée pour déformer la deuxième partie d'isolation (74) vers la première électrode (54) dans la position fermée.

6. Instrument électrochirurgical selon l'une quelconque des revendications 1 à 3, dans lequel la seconde mâchoire (36) inclut une partie en saillie (86) et une partie en retrait (88), dans lequel, dans la position fermée, la partie en saillie (86) est en contact avec la première surface (50) et la partie en retrait (88) est espacée de la première électrode (54), et dans lequel la partie en saillie (86) est flexible de sorte que la seconde mâchoire (36) peut être déplacée davantage vers la première surface (50) par rapport à la position fermée de sorte que la partie en retrait (88) peut être rapprochée de la première électrode (54).

7. Instrument électrochirurgical selon l'une quelconque des revendications précédentes, dans lequel la deuxième électrode (56) recouvre la première électrode (54) sur le côté de la première électrode (54) orienté à l'opposé de la première surface (50) et/ou dans lequel la première partie d'isolation (58) est agencée entre la première électrode (54) et la deuxième électrode (56).

8. Instrument électrochirurgical selon l'une quelconque des revendications précédentes, dans lequel une partie de la deuxième électrode (56) est en forme de plaque et inclut des faces d'extrémité, dans lequel les faces d'extrémité forment des sections de la deuxième électrode (56) qui sont exposées sur la première surface (50), dans lequel, dans une vue en coupe transversale de la première mâchoire (34), les parties de la deuxième électrode (56) sont en forme de U ou en forme de V,
dans lequel facultativement les sections de la deuxième électrode (56) qui sont exposées sur la première surface (50) s'étendent au moins partiellement parallèlement les unes aux autres et/ou avec la première électrode (54) agencées entre celles-ci.

9. Instrument électrochirurgical selon l'une quelconque des revendications 1 à 6, dans lequel une partie de la deuxième électrode (56) est intégrée dans la première partie d'isolation (58), dans lequel facultativement une partie d'extrémité distale de la deuxième électrode (56) est exposée sur la première partie d'isolation (58).

10. Instrument électrochirurgical selon l'une quelconque des revendications précédentes, dans lequel la première mâchoire (34) inclut une face d'extrémité distale (68), dans lequel la première électrode (54), la deuxième électrode (56) et/ou la première partie d'isolation (58) sont exposées sur la face d'extrémité distale (68).

11. Instrument électrochirurgical selon l'une quelconque des revendications précédentes, comprenant en outre une articulation (32) présentant un axe de pivot et un bras (70) mobile par rapport à la seconde mâchoire (36),
dans lequel le bras (70) est configuré pour être déplacé entre une position de pression, dans laquelle le bras (70) et la première surface (50) sont rapprochés pour presser le tissu entre eux, et une position rétractée, dans laquelle le bras (70) est espacé de la première surface (50),
dans lequel facultativement la seconde mâchoire (36) peut tourner autour d'un axe de pivot de l'articulation (32), dans lequel le bras (70) peut également tourner autour de l'axe de pivot, et
dans lequel en outre facultativement
la seconde mâchoire (36) et le bras (70) sont configurés pour être tournés indépendamment l'un de l'autre, ou
la seconde mâchoire (36) inclut une fente de mâchoire (84) et le bras (70) inclut une came de bras (86), dans lequel la came de bras (86) est insérée dans la fente de mâchoire (84) pour fournir un mouvement du bras (70) par rapport à la seconde mâchoire (36) entre la position rétractée et la position de pression.

12. Instrument électrochirurgical selon la revendication 11, dans lequel la seconde mâchoire (36) inclut une ouverture à travers laquelle le bras (70) peut être déplacé, et/ou
dans lequel le bras (70) inclut une deuxième partie d'isolation (74) qui est configurée pour être en contact avec la première électrode (54) dans la position de pression du bras (70), et/ou
dans lequel le bras (70) et la seconde mâchoire (36) incluent respectivement des matériaux conducteurs qui forment la troisième électrode (66), et/ou
la seconde mâchoire (36) inclut une troisième partie d'isolation qui recouvre l'ouverture et est flexible à travers l'ouverture de sorte que le bras (70) est configuré pour déformer la troisième partie d'isolation dans la position fermée ou le bras (70) déforme la troisième partie d'isolation dans une position de pression du bras (70), dans laquelle le bras (70) et la première surface (50) sont rapprochés pour couper du tissu entre eux, dans lequel facultativement la troisième partie d'isolation est réalisée en silicium.

13. Instrument électrochirurgical selon l'une quelconque des revendications précédentes, dans lequel la première partie d'isolation (58) inclut une première partie de matériau (102) et une seconde partie de matériau (104), dans lequel la première partie de matériau (102) est agencée entre la ligne de transmission et la seconde partie de matériau (104), et dans lequel un matériau de la première partie de matériau (102) est différent d'un matériau de la seconde partie de matériau (104).

14. Appareil électrochirurgical pour refermer et couper un tissu, comprenant
une unité de génération (14) pour générer de l'énergie électromagnétique radiofréquence et/ou hyperfréquence, et
un instrument électrochirurgical (12) selon l'une quelconque des revendications précédentes,
dans lequel la ligne de transmission est configurée pour transporter l'énergie électromagnétique radiofréquence et/ou hyperfréquence à partir de l'unité de générateur (14) vers la première électrode (54), la deuxième électrode (56) et/ou la troisième électrode (66).

15. Appareil électrochirurgical selon la revendication 14, dans lequel l'unité de générateur (14) est configurée pour générer simultanément ou alternativement une énergie électromagnétique hyperfréquence d'une première fréquence et une énergie électromagnétique radiofréquence d'une seconde fréquence.
